(19) **Europäisches Patentamt European Patent Office Office européen des brevets**

(11) **EP 4 499 035 B1**

(12) **FASCICULE DE BREVET EUROPEEN**

(45) Date de publication et mention de la délivrance du brevet:
**17.12.2025 Bulletin 2025/51**

(21) Numéro de dépôt: **23716677.2**

(22) Date de dépôt: **23.03.2023**

(51) Classification Internationale des Brevets (IPC):
***A61K 8/9789*** *(2017.01)* ***A61Q 19/00*** *(2006.01)*
***A61Q 19/08*** *(2006.01)*

(52) Classification Coopérative des Brevets (CPC):
**A61K 8/9789; A61Q 19/00; A61Q 19/005; A61Q 19/007; A61Q 19/08**

(86) Numéro de dépôt international:
**PCT/IB2023/052867**

(87) Numéro de publication internationale:
**WO 2023/180981 (28.09.2023 Gazette 2023/39)**

(54) **EXTRAIT DE MELALEUCA ALTERNIFOLIA ET UTILISATIONS COSMETIQUES DE CELUI-CI**

MELALEUCA ALTERNIFOLIA-EXTRAKT UND KOSMETISCHE VERWENDUNGEN DAVON

MELALEUCA ALTERNIFOLIA EXTRACT AND COSMETIC USES THEREOF

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **24.03.2022 FR 2202650**

(43) Date de publication de la demande:
**05.02.2025 Bulletin 2025/06**

(73) Titulaire: **Lucas Meyer Cosmetics**
**91300 Massy (FR)**

(72) Inventeurs:
• **ATTIA, Joan**
**31410 LAVERNOSE LACASSE (FR)**
• **DUROUX, Romain**
**31270 CUGNAUX (FR)**

(74) Mandataire: **Cabinet Becker et Associés**
**25, rue Louis le Grand**
**75002 Paris (FR)**

(56) Documents cités:
**FR-A1- 2 748 204 US-A1- 2010 092 398**
**US-A1- 2013 078 301**

EP 4 499 035 B1

**Description**

DOMAINE TECHNIQUE

La présente invention se rapporte au domaine cosmétique, en particulier aux agents cosmétiques pour lutter contre les effets de la fatigue sur la peau.

ARRIERE-PLAN TECHNOLOGIQUE

La peau est la première barrière protégeant l'organisme des agressions extérieures. Cet organe est composé par plusieurs couches de tissu. On distingue l'épiderme qui est la partie la plus externe de la peau, le derme, un tissu conjonctif constitué de fibroblastes et d'une matrice extracellulaire, qui assure les fonctions de cohésion et de nutrition de la peau, et l'hypoderme constitué d'adipocytes.

L'épiderme est constitué par plusieurs strates cellulaires de kératinocytes. On distingue, entre autres, la couche germinative de l'épiderme, appelée couche basale, contenant, notamment, les cellules souches cutanées, la couche épineuse, *Stratum spinosum,* constituée de plusieurs couches de cellules polygonales, la couche granuleuse, *Stratum granulosum,* comprenant une à trois couches de cellules aplaties contenant des inclusions cytoplasmiques, les grains de kératohyaline, et enfin, le couche cornée, *Stratum corneum* qui est composée de cellules anucléées et riches en kératine appelées cornéocytes qui correspondent au stade terminal de différenciation des kératinocytes.

Les cellules les plus externes de la couche cornée sont continuellement éliminées et remplacées par les cellules d'une couche inférieure, selon un processus appelé desquamation. La régénération cellulaire de la couche cornée est basée sur un processus de maturation cellulaire dans lequel les cellules de la couche basale de l'épiderme se différencient et migrent progressivement à travers les différentes strates de l'épiderme jusqu' à arriver à la couche cornée sous la forme de cornéocytes.

La peau est généralement la première à imprimer les signes de fatigue. La fatigue peut être causée par de nombreux facteurs, tels que le manque de sommeil, une hygiène alimentaire mauvaise ou déséquilibrée, le surmenage, une activité intense ou inadaptée au travail, ou des perturbations dans la sphère familiale. Les femmes menant de front vie de famille et travail, et les jeunes actifs en milieu urbain sont particulièrement exposés à une fatigue chronique.

Les individus exposés à une telle fatigue peuvent présenter des traits tirés, une perte d'éclat du teint voire un teint brouillé, des cernes et des poches au niveau des yeux. En d'autres termes, leur visage paraît fatigué et marqué, voire maladif. Or, renvoyer l'image d'une personne en forme peut être une préoccupation majeure pour les individus actifs travaillant, notamment dans un milieu concurrentiel et/ou au contact des clients.

Néanmoins, il reste, à l'heure actuelle, un besoin pour de nouveaux actifs cosmétiques pour prévenir ou traiter les effets visibles de la fatigue sur la peau. Le document FR 2 748 204 divulgue l'utilisation en cosmétique d'un extrait huileux rectifié de Melaleuca alternifolia comprenant au moins 60 % de terpinèn-4-ol.

RESUME DE L'INVENTION

L'invention a pour objet l'utilisation cosmétique d'un extrait huileux de *Melaleuca alternifolia,* en tant qu'agent cosmétique régénérateur, réparateur ou anti-fatigue de la peau saine, dans laquelle ledit extrait comprend :

- plus de 20% de composés choisis parmi les sesquiterpènes et sesquiterpénoïdes, et
- moins de 6 % de terpinèn-4-ol,

les pourcentages étant exprimés par rapport à la teneur totale de composés volatils présents dans l'extrait déterminée par chromatographie en phase gazeuse couplée à un détecteur à ionisation de flamme (GC/FID).

De préférence, les pourcentages correspondent au pourcentage d'aire des pics correspondant aux composés chimiques d'intérêt par rapport à l'aire totale des pics du chromatogramme obtenu par analyse GC/FID sur colonne apolaire.

De préférence, ledit extrait de *Melaleuca alternifolia* comprend moins de 15 %, de préférence moins de 10%, mieux encore moins de 2,0% de terpinéols. En particulier, l'extrait *de Melaleuca alternifolia* selon l'invention peut comprendre :

- moins de 5,0 %, de préférence moins de 2,0%, mieux moins de 1,0%, de terpinèn-4-ol, et/ou
- moins de 5,0 %, de préférence moins de 2,0%, mieux moins de 1,5%, d'alpha-terpinéol. Dans certains modes de réalisation, ledit extrait *de Melaleuca alternifolia* comprend plus de 30 %, de préférence plus de 40% de composés choisis parmi les sesquiterpènes et sesquiterpénoïdes. En particulier, ledit extrait de *Melaleuca alternifolia* peut comprendre :

- de 5 à 15 % d'aromadendrène,
- de 10 à 25 % de lédène,
- de 10 à 25% de delta-cadinène,
- de 1 à 5 % de globulol, et
- de 1 à 5 % de viridifloral.

Dans un mode particulier de réalisation, l'extrait de *Melaleuca alternifolia* est obtenu par distillation fractionnée d'une huile essentielle de feuilles et/ou de rameaux terminaux de *Melaleuca alternifolia.*

Dans certains modes de réalisation, ledit extrait de *Melaleuca alternifolia* est utilisé pour traiter ou prévenir un ou plusieurs signes de la fatigue cutanée choisis parmi une perte d'éclat de la peau, un teint terne, un teint brouillé, une perte d'uniformité du teint, les traits du visage tirés, des cernes au niveau des yeux, des poches au niveau des yeux, les paupières gonflées, une altération de l'aspect lisse de la peau, une augmentation de la rugosité de la peau, une perte d'élasticité, une perte de densité, une perte de fermeté, une perte d'hydratation, une apparition de rides, une apparition de rougeurs, et leurs combinaisons.

Dans d'autres modes de réalisation, ledit extrait de *Melaleuca alternifolia* est utilisé en tant qu'agent cosmétique pour favoriser ou améliorer la récupération cutanée pendant le sommeil et/ou en tant qu'agent cosmétique pour potentialiser les mécanismes naturels de réparation et de régénération de la peau contrôlés par la mélatonine.

Ledit extrait de *Melaleuca alternifolia* selon l'invention peut en outre être utilisé pour augmenter la durée totale de sommeil, et/ou celle du sommeil profond.

Typiquement, ledit extrait de *Melaleuca alternifolia* est présent en tant qu'agent actif cosmétique dans une composition cosmétique, de préférence une crème, un baume, un masque, un sérum ou une lotion.

L'invention a également pour objet un extrait de *Melaleuca alternifolia* tel que décrit ci-avant. Un objet supplémentaire est une composition précurseur pour la préparation d'une composition cosmétique comprenant de 0,1% à 1% en poids, de préférence de 0,1 à 0,5% en poids, d'un extrait de *Melaleuca alternifolia* selon l'invention, dans un véhicule cosmétiquement acceptable, de préférence le pentylène glycol ou le triheptanoïne.

Un autre objet supplémentaire est un ingrédient cosmétique comprenant de 0,1% à 1% en poids, de préférence de 0,1 à 0,5% en poids, d'un extrait de *Melaleuca alternifolia* selon l'invention, dans un véhicule cosmétiquement acceptable, de préférence le pentylène glycol ou le triheptanoïne. Ledit ingrédient cosmétique est de préférence utilisé en tant qu'agent cosmétique régénérateur, réparateur ou anti-fatigue de la peau saine.

L'invention a également pour objet une composition cosmétique comprenant l'extrait de *Melaleuca alternifolia,* la composition précurseur, ou l'ingrédient cosmétique selon l'invention, en combinaison avec au moins excipient acceptable sur le plan cosmétique.

La composition cosmétique selon l'invention peut comprendre de 0,0005 à 0,01%, de préférence de 0,001 % à 0,005 % en poids dudit extrait de *Melaleuca alternifolia.* Alternativement, la composition cosmétique selon l'invention peut comprendre de 0,5 % à 5 % en poids, de préférence de 1 % à 3% en poids, de l'ingrédient cosmétique ou de la composition précurseur.

La composition cosmétique selon l'invention peut être choisie dans le groupe constitué par les solutions aqueuses, les solutions hydroalcooliques, les émulsions huile-dans-eau (H/E) ou eau-dans huile (E/H) ou multiple (triple : E/H/E ou H/E/H), les nanoémulsions, en particulier des nanoémulsions H/E, les gels aqueux, ou les dispersions d'une phase grasse dans une phase aqueuse à l'aide de sphérules, les suspensions, de préférence en milieux aqueux ou hydroalcoolique, les suspensions de liposomes, les poudres, les lotions, laits, les crèmes, les onguents, les gels, les mousses, et les pommades.

La composition cosmétique selon l'invention peut être un soin pour traiter ou prévenir la fatigue cutanée, par exemple un sérum, une crème, un masque ou un baume, de préférence de nuit. L'invention a pour objet un procédé cosmétique pour prévenir ou traiter signes de la fatigue cutanée chez un individu, ledit procédé comprenant l'application d'une quantité cosmétiquement efficace d'un extrait de *Melaleuca alternifolia* ou d'une composition cosmétique selon l'invention sur la peau.

Enfin, l'invention a pour objet l'utilisation d'un extrait de *Melaleuca alternifolia* ou d'un ingrédient cosmétique tel que décrit dans la présente demande dans la fabrication d'une composition cosmétique pour traiter, prévenir ou atténuer les signes de la fatigue cutanée. Chacun des aspects et modes de réalisation décrits ici est susceptible d'être utilisé ensemble, sauf si ceci est exclu explicitement ou exclu clairement du contexte du mode de réalisation ou de l'aspect considéré.

FIGURES

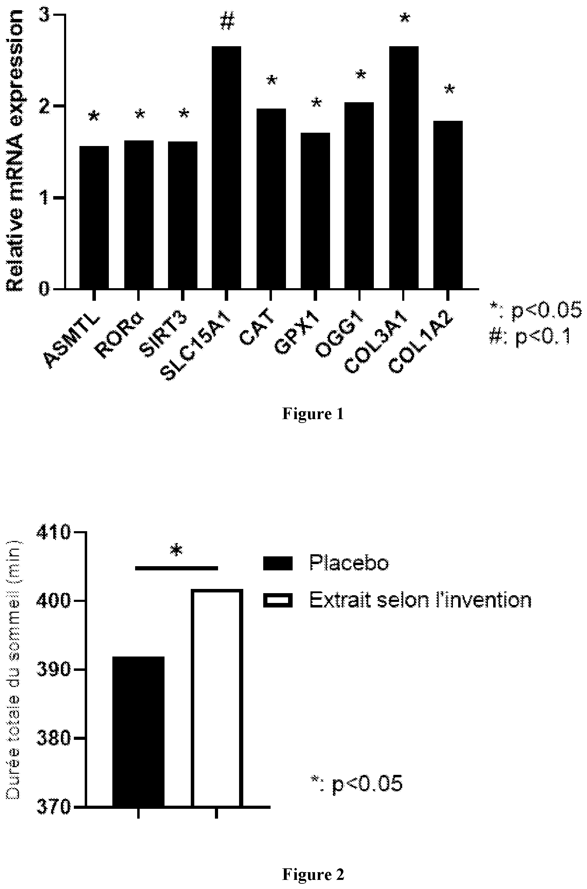

La Figure 1 représente la modulation de différents gènes associés à la réparation de la peau ainsi qu'à la voie de signalisation de la mélatonine en présence de l'extrait selon l'invention chez des explants de peau soumis à un stress UV par rapport à des explants placebo (soumis à un stress UV, en absence de l'extrait selon l'invention).

La Figure 2 montre l'effet de l'application cutanée de l'extrait selon l'invention au niveau du visage sur la durée totale du sommeil.

La Figure 3 montre l'effet de l'application cutanée de l'extrait selon l'invention au niveau du visage sur la durée du sommeil profond.

Les Figures 4 à 10 montrent les résultats de l'essai clinique de l'Exemple 6.

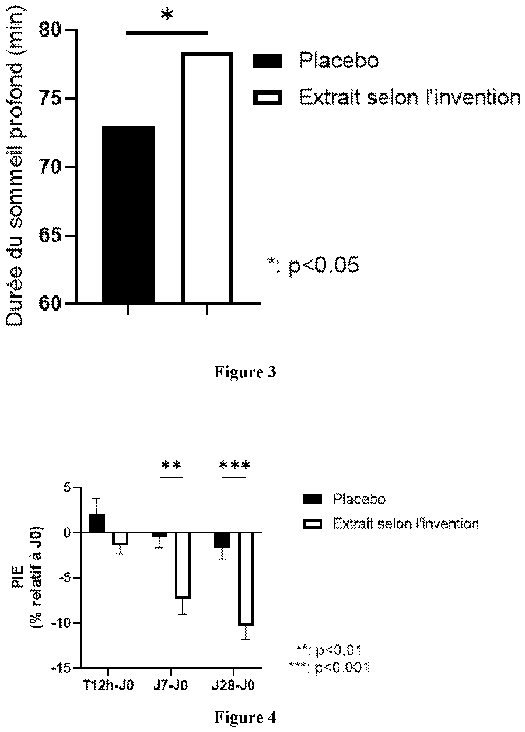

La Figure 4 montre la perte insensible en eau (PIE) pour le groupe traité avec la crème « actif » (Extrait selon l'Invention) et le groupe traité avec la crème placebo (Placebo) à T 12h, J 7 et J 28 en pourcentage relatif par rapport à J0.

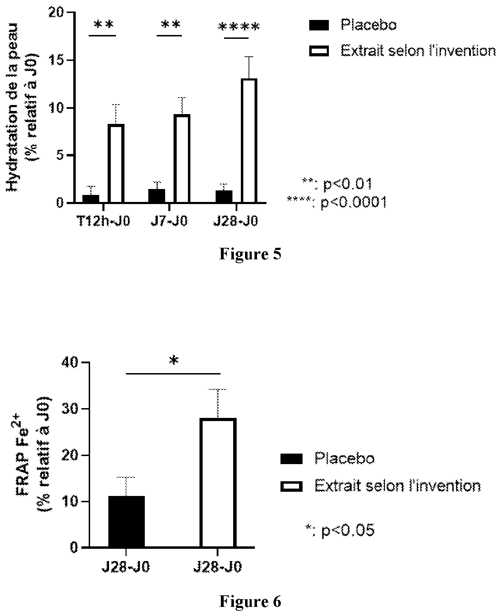

La Figure 5 montre l'hydratation de la peau évaluée par cornéométrie pour le groupe traité avec la crème « actif » (Extrait selon l'Invention) et le groupe traité avec la crème placebo (Placebo) à T 12h, J 7 et J 28 en pourcentage relatif par rapport à J0.

La Figure 6 montre le potentiel antioxydant de la peau mesuré par le test FRAP (Ferric Reducing Antioxidant Parameter) à partir d'échantillons des premières couches du *stratum corneum* prélevés à J0 et J28 sur les joues à l'aide d'un patch adhésif de type Corneofix® pour le groupe traité avec la crème « actif » (Extrait selon l'Invention) et le groupe traité avec la crème placebo (Placebo) à J28 en pourcentage relatif par rapport à J0.

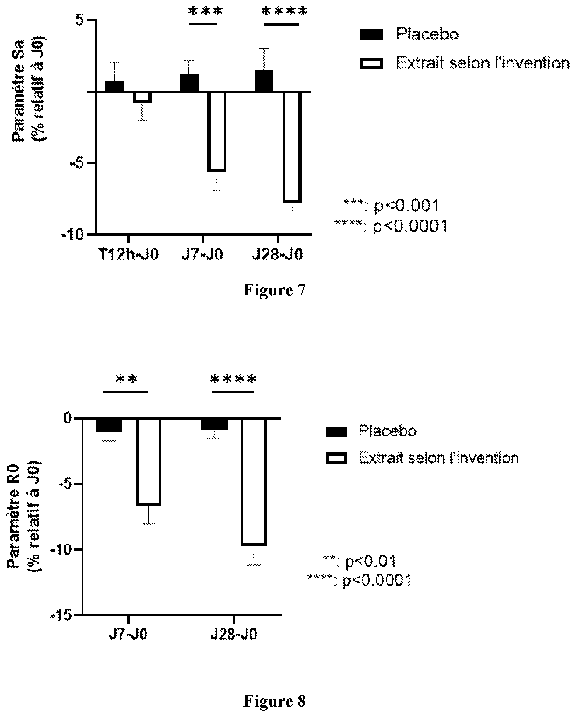

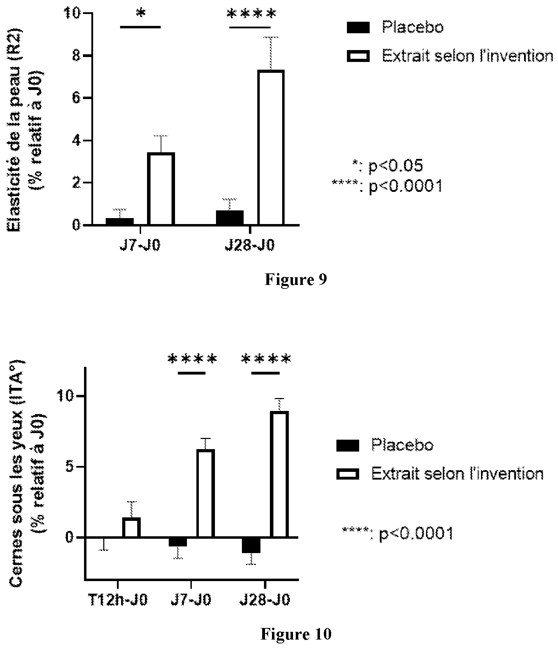

Les Figures 7, 8 et 9 montrent les résultats des expériences visant à évaluer l'aspect lisse et les propriétés biomécaniques de la peau. La Figure 7 montre l'évolution du paramètre Sa (aspect lisse de la peau) à T12h, J7 et J28 en pourcentage relatif rapport à J0. Les Figures 8 et 9 montrent les paramètres R0 (fermeté) et R2 (élasticité) en pourcentage relatif par rapport à J0 pour le groupe traité avec la crème « actif » (Extrait selon l'Invention) et le groupe traité avec la crème placebo.

La Figure 10 montre les résultats d'évaluation des cernes à T12h, J7 et J28 en pourcentage relatif rapport à J0 pour le groupe traité avec la crème « actif » (Extrait selon l'Invention) et le groupe traité avec la crème placebo.

Analyse statistique : *$p<0,05$ ; **$p<0,01$ ; ***$p<0,001$ ; ****$p<0.0001$.

DESCRIPTION DETAILLEE

La mélatonine est une hormone produite par la glande pinéale (également appelée épiphyse). La sécrétion de mélatonine est inhibée en présence de lumière et stimulée lorsqu'il fait sombre. Par l'intermédiaire de la mélatonine, la glande pinéale informe le cerveau sur les durées relatives des heures d'obscurité et d'éclairage sur une période de 24 h (cycle journalier), mais aussi pendant toute l'année (cycle saisonnier). La mélatonine est appelée « l'hormone du sommeil » et joue un rôle central dans la régulation des rythmes chronobiologiques. Cette hormone est produite dans différents tissus. Le procédé commence avec le Tryptophane qui est convertit en sérotonine *via* différentes enzymes pour finalement transformer cette dernière en mélatonine par action successive de la Arylalkylamine N-acétyltransferase et de la hydroxyindole-O-methyltransferase. La mélatonine peut agir sur la peau par deux voies différentes : de façon indépendante et dépendante des récepteurs. La mélatonine est capable d'assurer un rôle d'antioxydant cellulaire, notamment en piégeant directement les radicaux libres, mais également de manière indirecte en augmentant l'activité d'enzymes antioxydantes. Cette molécule qui a le potentiel de franchir les membranes cellulaires, agit également pour maintenir et protéger l'activité mitochondriale.

La mélatonine présente aussi des effets bénéfiques par activation des récepteurs à la mélatonine. Les récepteurs à la mélatonine sont des récepteurs couplés à la protéine G qui existent sous deux sous-types chez l'homme, à savoir MT1 et MT2. Ces deux récepteurs créent un rythme biologique qui induit le sommeil. Les récepteurs à la mélatonine sont exprimés au niveau du cerveau mais également au niveau périphérique. Le récepteur MT1 est exprimé notamment au niveau de la peau. Le récepteur MT1 est impliqué dans les mécanismes de protection de la peau contre le stress environnemental mais également dans la pigmentation ou le vieillissement cutané.

Enfin, la mélatonine interagit avec le récepteur nucléaire ROR$\alpha$ qui agit comme un facteur de transcription important impliqué dans de nombreux mécanismes biologiques, notamment antioxydant.

La Demanderesse a conçu un extrait particulier de *Melaleuca alternifolia* caractérisé par une composition originale, à savoir une faible teneur en terpinè-4-ol et une teneur élevée en composés de type sesquiterpènes et sesquiterpénoïdes. Cet extrait est issu de la distillation fractionnée d'une huile essentielle traditionnelle de *Melaleuca alternifolia.* De manière étonnante, la Demanderesse a montré que cet extrait possède des activités biologiques permettant d'agir sur la voie de signalisation de la mélatonine pour mimer et renforcer son activité. La Demanderesse a notamment montré que l'extrait selon l'invention est capable d'activer des gènes spécifiques de la voie de la signalisation à la mélatonine dans des explants de peau soumis à une exposition UV, tels que ASMTL, ROR$\alpha$, SIRT3 et SLC15A1. ASMTL étant une enzyme permettant la synthèse de la mélatonine et SLC15A1 étant un transporteur de la mélatonine au niveau mitochondrial, l'extrait possède donc la capacité de potentialiser l'effet de la mélatonine. De plus, comme la mélatonine, l'extrait est capable d'activer RORα et SIRT3 qui jouent un rôle prépondérant face aux dommages cellulaires en limitant le stress oxydatif. De plus, l'extrait a induit l'expression d'enzymes antioxydantes telles que CAT et GPX1 L'extrait selon l'invention a été également capable d'induire des gènes impliqués dans la synthèse du collagène (COL3A1 et COL1A2) et de réparation de l'ADN (OGG1) pour favoriser les mécanismes naturels de régénération et de réparation de la peau contrôlés par la mélatonine. De manière tout à fait surprenante, la Demanderesse a montré que l'application de l'extrait selon l'invention en une très faible concentration sur la peau permet d'améliorer la qualité du sommeil chez des individus présentant des troubles légers du sommeil dus à leur mode de vie, notamment en augmentant la durée totale du sommeil mais également la durée du sommeil profond, favorisant ainsi les mécanismes de réparation naturels se produisant notamment pendant cette phase du sommeil.

Enfin, la Demanderesse a confirmé l'effet antifatigue et régénérateur de l'extrait de *Melaleuca alternifolia* selon l'invention au cours d'un essai clinique réalisé chez des volontaires sains présentant des cycles de sommeil perturbés (Exemple 6). Cet essai clinique a montré que l'application journalière d'une crème comprenant l'extrait selon l'invention permet de prévenir et/ou de traiter les signes de la fatigue cutanée. L'Extrait selon l'Invention a notamment permis d'améliorer la fonction barrière de la peau, d'améliorer le pouvoir antioxydant de la peau, de prévenir et d'améliorer l'aspect lisse et les propriétés biomécaniques de la peau et de réduire les cernes chez les volontaires, et ceci de manière significative par rapport au groupe de volontaires traités avec la crème placebo. L'Extrait selon l'Invention a également permis de réduire la profondeur des rides au niveau des pattes d'oie, ce qui souligne un effet antiride.

Ainsi, l'ensemble de ces résultats soutient l'utilisation cosmétique par voie topique de l'extrait selon l'invention en tant qu'agent antifatigue, c'est-à-dire pour traiter ou prévenir les signes cutanés de la fatigue, et plus généralement en tant qu'agent pour favoriser ou améliorer la récupération de la peau pendant le sommeil, notamment en potentialisant les mécanismes naturels de réparation et de régénération de la peau contrôlés par la mélatonine.

Sans vouloir être liée par une quelconque théorie, la Demanderesse est d'avis que ces propriétés résultent de la composition spécifique de l'extrait préparé par distillation fractionnée de l'huile essentielle de *M. alternifolia.*

A la connaissance de la Demanderesse, l'extrait selon l'invention et son utilisation en tant qu'agent antifatigue cutané n'ont jamais été décrits ou suggérés dans l'art antérieur.

*Melaleuca alternifolia* est une plante endémique d'Australie que les Arborigènes utilisent traditionnellement en tant qu'herbe médicinale et agent antiseptique. L'art antérieur concerne essentiellement l'huile essentielle de *Melaleuca alternifolia,* qui présente une composition et des utilisations distinctes de l'extrait selon l'invention. En effet, l'huile essentielle *de Melaleuca alternifolia,* est une huile essentielle typiquement obtenue par entraînement à la vapeur de feuilles et/ou d'extrémités de rameaux de *Melaleuca alternifolia,* c'est -à-dire l'arbre à thé. Le composant actif principal de l'huile essentielle de *M. alternifolia* est le terpinèn-4-ol. A titre d'exemple, l'huile essentielle de *Melaleuca alternifolia* telle que définie selon la norme ISO 4730:2017 comprend, entre autres, de 35-48 % de terpinèn-4-ol, ainsi que 14-28% de gamma-terpinène, 6-12% d'alpha-terpinène, et 0,2-5% d'alpha-terpinéol.

L'huile essentielle de *M. alternifolia* est utilisée traditionnellement en dermatologie ou en médecine pour ses propriétés anti-acnéiques, antibactériennes et antifongiques. L'huile essentielle de *M. alternifolia* est ainsi utilisée sur la peau ou les muqueuses pour traiter des infections telles que des mycoses, des abcès cutanés, des furoncles ou encore des aphtes. Elle est également utilisée pour traiter certaines infections ORL ou encore en tant que répulsif en aromathérapie.

La présente invention a donc pour objet ledit extrait de *Melaleuca alternifolia* et ses utilisations dans le domaine cosmétique.

## a. Extrait de *Melaleuca alternifolia* selon l'invention

Ainsi, selon un premier aspect, la présente invention concerne un extrait de *Melaleuca alternifolia* pauvre en terpinèn-4-ol, voire plus généralement en terpinéols et en monoterpènes, et enrichi en sesquiterpènes et/ou sesquiterpénoïdes.

L'extrait de *Melaleuca alternifolia* selon l'invention est liposoluble. Plus exactement, l'extrait selon l'invention est un extrait huileux, également désigné sous le terme d'huile.

De préférence, l'extrait *de Melaleuca alternifolia* selon l'invention comprend :

- plus de 20% de composés choisis parmi les sesquiterpènes et sesquiterpénoïdes, et
- moins de 6 % de terpinèn-4-ol.

Sauf indication contraire, dans la présente demande, les pourcentages des composés compris dans l'extrait de *Melaleuca alternifolia* correspondent aux pourcentages d'aire des pics correspondant aux composés chimiques d'intérêt par rapport à l'aire totale des pics du chromatogramme obtenu par analyse GC/FID sur colonne apolaire, par exemple à base de diméthylpolysiloxane, de préférence réalisée selon les conditions décrites dans l'exemple 1.

Dans certains modes de réalisation, l'extrait de *Melaleuca alternifolia* selon l'invention comprend moins de 5,0 %, par exemple moins de 4,5%, 4,0% ou 3,5 % de terpinèn-4-ol. Dans certains modes de réalisation, l'extrait selon l'invention comprend moins de 3,0 %, par exemple moins de 2,0% ou de 1,0% voire moins de 0,5 % de terpinèn-4-ol.

Le terpinèn-4-ol est également appelé le 4-terpinéol, para-menth-1-én-4-ol, ou 1-isopropyl-4-méthyl-3-cyclohexèn-1-ol.

Dans certains modes de réalisation, l'extrait de *Melaleuca alternifolia* selon l'invention comprend moins de 5,0 %, de préférence moins de 2,0%, voire moins de 1,5%, d'alpha-terpinéol.

Dans certains modes de réalisation, l'extrait de *Melaleuca alternifolia* selon l'invention comprend, d'une manière générale, moins de 15,0 %, de préférence moins de 10,0%, voire moins de 8,0%, 6,0%, 5,0%, 4,0%, 3,0% ou de 2,0% de terpinéols.

Les terpinéols sont des alcools monoterpéniques monocycliques insaturés. Dans le cadre de la présente invention, les terpinéols comprennent le terpinèn-4-ol, l'alpha-terpinéol, le béta-terpinéol, le gamma-terpinéol, le terpinèn-1-ol, et le terpinén-5-ol.

Dans certains modes de réalisation, l'extrait de *Melaleuca alternifolia* selon l'invention comprend moins de 10 %, de préférence moins de 5% ou de 2%, voire moins de 1% de monoterpènes.

Les monoterpènes sont des terpènes constitués formellement de deux unités isoprène.

Dans le cadre de la présente invention, les monoterpènes comprennent, entre autres, les terpinènes tels que l'alpha-terpinène et le gamma-terpinène (ou « terpinolène »), l'alpha-phellandrène, l'alpha-p-diméthylstyrène, l'alpha-pinène, le beta-pinène, le limonène, le sabinène, le para-cymène, l'alpha-thujène, ou le myrcène,.

En particulier, l'extrait de *Melaleuca alternifolia* selon l'invention peut comprendre moins de 5%, de préférence moins de 2%, voire moins de 1% ou de 0,5% de terpinènes.

Dans certains modes de réalisation, l'extrait de *Melaleuca alternifolia* selon l'invention comprend moins de 10 %, de préférence moins de 5% ou de 2%, voire moins de 1% de monoterpénoïdes autres que les terpinéols, tels que l'eucalyptol, le camphre, ou le trans-pipéritol.

Dans certains modes de réalisation, l'extrait de *Melaleuca alternifolia* selon l'invention comprend plus de 30% ou de 40%, voire plus de 50%, de 60%, ou de 70% de composés choisis parmi les sesquiterpènes et sesquiterpénoïdes.

Les sesquiterpènes sont des terpènes constitués formellement de trois unités isoprène.

Dans le cadre de la présente invention, les sesquiterpènes comprennent, entre autres, l'aromadendrène, le lédène (ou « viridiflorène »), le delta-cadinène, le beta-caryophyllène, l'alloaromadendrène, le valencène, le beta-sélinène, le bicycloélémène, l'alpha-cubébène, le beta-guaiène, l'alpha-copaène, l'alpha-gurjunène, le beta-ylangène, l'alpha-humulène, ou le beta-cédrène, le beta-maaliène, le gamma murolène, le gamma-curcumène, le gamma-cadinène, ou le cadina-1,4-diène.

Les sesquiterpénoïdes sont des dérivés des sesquiterpènes, généralement formés par modification synthétique ou biologique des sesquiterpènes (typiquement, par oxydation et/ou réarrangement).

En particulier, les sesquiterpénoïdes comprennent des groupes fonctionnels additionnels par rapport aux sesquiterpènes, notamment un oxygène (par exemple une fonction ester, alcool, cétone ou encore étheroxyde) et/ou des groupes alkyles en moins (par exemple un méthyle en moins).

Dans le cadre de la présente invention, les sesquiterpénoïdes comprennent, entre autres, le calaménène, l'alpha-calacorène, le beta-calacorène, le delta-cadinol, l'éléma-1,11-dién-15-al, le globulol, le vidifloral, le cadin-4-en-10-ol, le lédol, le rosifoliol, ou le spathulénol. L'extrait selon l'invention peut notamment comprendre au moins 20% (e.g. au moins 25%) des composés sesquiterpènes et sesquiterpénoïdes suivant : l'aromadendrène, le lédène, le delta-cadinène, le globulol et le viridifloral.

Dans un mode de réalisation particulier, ledit extrait de *Melaleuca alternifolia* selon l'invention comprend :

- de 5 à 15 % d'aromadendrène,
- de 10 à 25 % de lédène, et
- de 10 à 25% de delta-cadinène.

Dans un mode de réalisation plus particulier, ledit extrait de *Melaleuca alternifolia* selon l'invention comprend :

- de 5 à 15 % d'aromadendrène,
- de 10 à 25 % de lédène,
- de 10 à 25% de delta-cadinène,
- de 1,0 à 5,0 % de globulol, et
- de 1,0 à 5,0 % de viridifloral.

Dans un autre mode de réalisation supplémentaire, l'extrait selon l'invention comprend :

- moins de 6 % (par exemple moins de 5 %, de 4,5%, de 4,0%, de 3,5 %, de 3,0 %, de 2,0%, de 1%, de 0,5 %) de terpinèn-4-ol,
- moins de 5 %, (par exemple moins de 2,0% ou de 1,5%) d'alpha-terpinéol,
- moins de 15,0 % (par exemple moins de 10,0%, de 8,0%, de 6,0%, de 5,0%, de 4,0%, de 3,0% ou de 2,0%) de terpinéols,
- moins de 10 % (par exemple moins de 5%, de 2%, ou de 1%) de monoterpènes,
- moins de 10 % (par exemple moins de 5%, de 2%, ou de 1%) de monoterpénoïdes autres que les terpinéols,
- au moins 25% (par exemple plus de 30%, de 40%, de 50%, de 60%, ou de 70%) de composés choisis parmi les sesquiterpènes et sesquiterpénoïdes.

De préférence, l'extrait selon l'invention comprend au moins 25% de composés choisis parmi l'aromadendrène, le lédène et le delta-cadinène.

En particulier, l'extrait selon l'invention peut comprendre :

- de 5à 15 % d'aromadendrène,
- de 10 à 25 % de lédène, et
- de 10 à 25% de delta-cadinène.

Dans un autre mode de réalisation supplémentaire, l'extrait selon l'invention comprend :

- moins de 6 % (par exemple moins de 5 %, de 4,5%, de 4,0%, de 3,5 %, de 3,0 %, de 2,0%, de 1%, de 0,5 %) de terpinèn-4-ol,
- moins de 5 %, (par exemple moins de 2,0% ou de 1,5%) d'alpha-terpinéol,
- moins de 15,0 % (par exemple moins de 10,0%, de 8,0%, de 6,0%, de 5,0%, de 4,0%, de 3,0% ou de 2,0%) de terpinéols,
- moins de 10 % (par exemple moins de 5%, de 2%, ou de 1%) de monoterpènes,
- moins de 10 % (par exemple moins de 5%, de 2%, ou de 1%) de monoterpénoïdes autres que les terpinéols,
- au moins 27 % (par exemple plus de 30%, de 40%, de 50%, de 60%, ou de 70%) de composés choisis parmi les sesquiterpènes et sesquiterpénoïdes.

De préférence, l'extrait selon l'invention comprend au moins 27% de composés choisis parmi l'aromadendrène, le lédène, le delta-cadinène, le globulol et le viridifloral.

En particulier, l'extrait selon l'invention peut comprendre :

- de 5 à 15 % d'aromadendrène,
- de 10 à 25 % de lédène,
- de 10 à 25% de delta-cadinène,
- de 1,0 à 5,0 % de globulol, et
- de 1,0 à 5,0 % de viridifloral.

L'extrait selon l'invention peut être obtenu à partir d'une huile essentielle de *Melaleuca alternifolia* (dénommée ci-après « première huile essentielle » ou « huile essentielle de départ ») par distillation fractionnée.

L'huile essentielle de départ est typiquement riche en terpinèn-4-ol et est généralement obtenue par entraînement à la vapeur ou par hydrodistillation de tout ou partie *de Melaleuca alternifolia,* de préférence des feuilles et/ou des rameaux.

Dans certains modes de réalisation, l'huile essentielle de départ de *Melaleuca alternifolia* peut comprendre :

- de 35 à 48 % (par exemple de 37 à 45 %) de terpinèn-4-ol,
- de 10 à 28 % (par exemple de 14 à 28 %) de gamma-terpinène,
- de 5,0 à 13 % (par exemple de 6 à 12 %) d'alpha-terpinène,
- de 0,2 à 8,0 % (par exemple de 0,2 à 5,0 % ou de 1,5 à 8,0 %) d'alpha-terpinéol, et

De préférence, elle comprend moins de 3,0 % d'aromadendrène, moins de 3,0 % de lédène, moins de 3,0 % delta-cadinène, moins de 1,0 % de globulol, et moins de 1,0 % de viridifloral. L'extrait de *Melaleuca alternifolia* selon l'invention est avantageusement une fraction issue de la distillation fractionnée d'une huile essentielle de *Melaleuca alternifolia* . La distillation fractionnée est typiquement mise en œuvre sous pression réduite, à l'aide d'un dispositif de distillation fractionnée comprenant un bouilleur, une colonne de fractionnement, un condenseur et un récolteur. L'huile essentielle est mise à chauffer lentement sous pression réduite de manière à récolter différentes fractions successives, chaque fraction correspondant à un palier de température. La distillation fractionnée est réalisée de manière à récolter dans un premier temps au moins une (de préférence 2, 3 ou 4) fractions de distillation enrichies en composés de type monoterpènes et monoterpénoïdes (notamment le terpin-4-ol). Une fois la ou les fractions enrichies en composés de type monoterpènes et monoterpénoïdes récoltée(s), la fraction correspondant à l'extrait selon l'invention peut être récoltée. Cette fraction est typiquement obtenue dans des conditions de température et de pression réduite permettant de récolter des composés présentant une température d'ébullition supérieure à 200°C à pression atmosphérique.

La distillation fractionnée étant un procédé bien connu, l'homme du métier sait déterminer la température de chauffage et la pression à utiliser pour obtenir, à partir d'une huile essentielle donnée, une fraction correspondant à un extrait selon l'invention par e.g. par la mise en œuvre de tests de routine et/ou à l'aide d'abaques.

Dans un mode de réalisation préféré, l'extrait selon l'invention est une fraction de distillation d'une huile essentielle de *Melaleuca alternifolia,* de préférence obtenue par distillation fractionnée.

Ainsi, l'extrait selon l'invention peut être également désigné comme étant « une fraction de distillation d'une huile essentielle de *Melaleuca alternifolia »* ladite huile essentielle étant de préférence obtenue par entraînement à la vapeur ou hydrodistillation de feuilles et/ou de rameaux de *Melaleuca alternifolia.* L'extrait selon l'invention est donc typiquement un extrait huileux ou une huile de *Melaleuca alternifolia.* Comparativement à une huile essentielle classique, l'extrait selon l'invention est appauvri en monoterpènes et monoterpinoides.

Dans certains modes de réalisation, ledit extrait de *Melaleuca alternifolia* est obtenu par un procédé comprenant :

i) une étape d'hydrodistillation de tout ou partie de *Melaleuca alternifolia,* de préférence ses feuilles et/ou extrémités de rameaux, pour que soit obtenue une première huile essentielle,

ii) une étape de distillation fractionnée de ladite première huile essentielle dans des conditions de pression réduite et de température permettant de récolter une fraction correspondant audit extrait.

**b. Composition précurseur, Ingrédient cosmétique** et **composition cosmétique comprenant l'extrait selon l'invention**

L'extrait de *Melaleuca alternifolia* selon l'invention présente des effets cosmétiques et peut donc être utilisé en tant qu'agent cosmétique.

Dans le cadre de la présente invention, on entend par « *agent cosmétique »,* un agent exerçant un effet cosmétique sur la peau.

Dans le cadre de la présente invention, on entend par *« effet cosmétique »* tout effet non-thérapeutique, visant à modifier et/ou améliorer l'aspect de la peau ou des muqueuses comme les lèvres, ou à les protéger des agressions externes (soleil, vent, humidité, sécheresse, produits chimiques), par exemple à prévenir et/ou à corriger les phénomènes liés à leur vieillissement ou encore à prévenir ou à traiter les effets causés par la fatigue sur la peau.

Comme cela sera détaillé ci-après, l'extrait selon l'invention peut être utilisé en tant qu'agent cosmétique régénérateur, réparateur ou anti-fatigue de la peau. A cette fin, l'extrait de *Melaleuca alternifolia* selon l'invention est généralement introduit en tant qu'agent cosmétique dans une composition cosmétique. En d'autres termes, ledit extrait est typiquement appliqué sur la peau de l'individu à traiter par l'intermédiaire d'une composition cosmétique. Une composition précurseur ou un ingrédient cosmétique comprenant l'extrait de *Melaleuca alternifolia* selon l'invention peut être préalablement préparé(e) pour ensuite être incorporé(e) dans la composition cosmétique.

Ainsi, un objet de la présente invention est une composition précurseur pour la préparation d'une composition cosmétique, ladite composition précurseur comprenant de 0,1% à 1% en poids, de préférence de 0,1 à 0,5% en poids, de l'extrait de *Melaleuca alternifolia* selon l'invention, dans un véhicule cosmétiquement acceptable.

Un autre objet de la présente invention est un ingrédient cosmétique comprenant de 0,1% à 1% en poids, de préférence de 0,1 à 0,5% en poids, de l'extrait de *Melaleuca alternifolia* selon l'invention, dans un véhicule cosmétiquement acceptable

Le véhicule cosmétiquement acceptable peut être de tout type. Par exemple, il peut s'agir d'un solvant acceptable sur le plan cosmétique en particulier les alcools inférieurs notamment l'éthanol, l'isopropanol, le dipropylène glycol, le butylène glycol, le propanediol, la glycérine, le sorbitol, le propylène glycol, le pentylène glycol, la triheptanoïne, une solution aqueuse de ceux-ci, ou un mélange de ceux-ci.

De préférence, ledit véhicule est le pentylène glycol ou la triheptanoïne.

Dans un mode de réalisation particulier, l'ingrédient cosmétique ou la composition précurseur selon l'invention consiste essentiellement, ou consiste en un extrait de *Melaleuca alternifolia* selon l'invention dans un véhicule cosmétiquement acceptable choisi parmi le pentylène glycol et la tripheptanoïne.

L'extrait *de Melaleuca alternifolia* selon l'invention est de préférence présent en une teneur de 0,1% à 1% en poids, de préférence de 0,1 à 0,5% en poids, par rapport au poids total de l'ingrédient cosmétique.

Un autre objet de la présente invention est une composition cosmétique comprenant l'extrait de *Melaleuca alternifolia* selon l'invention, de préférence en tant qu'agent cosmétique, la composition précurseur ou l'ingrédient cosmétique selon l'invention, en combinaison avec au moins excipient acceptable sur le plan cosmétique.

Le ou les excipients acceptables sur le plan cosmétique présents dans la composition peuvent être choisis parmi les agents diluants, les agents dispersants, les agents gélifiants, les émollients, les agents de vectorisation (tels que les polymères polycationiques, les phospholipides, les liposomes unilamellaires ou multilamellaires, les niosomes, les éthosomes, les systèmes lamellaires, les nanosomes, les vésicules lipidiques ou polymériques, les nanosphères, les micro ou nano-particules de polymères naturels ou non, les hydrogels), les gommes, les résines, les solvants en particulier les alcools inférieurs notamment l'éthanol, l'isopropanol, le dipropylène glycol, le butylène glycol, le propanediol, la glycérine, le sorbitol, et le propylène glycol, les charges telles que les amidons modifiés et polymérisés, le dioxyde de titane, ou un stéarate métallique, les conservateurs, les huiles essentielles, les agents nacrés, les colorants, les absorbeurs d'odeur, les agents régulateurs du pH ou les agents neutralisants, les agents lubrifiants, les agents épaississants, les hydrotropes tels que les tensio-actifs dont les tensio-actifs anioniques, cationiques, amphotères ou non ioniques, les humectants, les agents mouillants, les agents stabilisateurs, les agents de charge, les agents dispersants, les parfums, les pigments organiques ou encore minéraux comme les oxydes de fer, les agents huileux comme les huiles ou des graisses d'origine végétale, les graisses d'origine animale, les huiles de synthèse comme la vaseline, les huiles de silicone (cyclométhicone), les esters d'alcool gras, des huiles fluorées, des cires, des argiles modifiées, des bentones, des sels métalliques d'acide gras, la silice, les polyéthylènes, du mica, les agents conservateurs, les agents antimicrobiens, des véhicules tels qu'une eau minérale, thermale ou florale, et/ou d'autres substances utilisés couramment en formulation dans le domaine cosmétique ou pharmaceutique.

Dans un mode de réalisation, la composition cosmétique comprend de 0,0005 à 0,01%, de préférence de 0,001 % à 0,005 % en poids de l'extrait de *Melaleuca alternifolia* selon l'invention.

Plus particulièrement, ladite composition cosmétique peut comprendre :

- de 0,0005% à 0,01% en poids, de préférence de 0,001 % à 0,005 % en poids dudit e de *Melaleuca alternifolia,* et
- de 99,99 % à 99,9995 % en poids d'un ou plusieurs excipients acceptables sur le plan cosmétique.

Dans un autre mode de réalisation, ladite composition cosmétique comprend de 0,5 % à 5 % en poids, de préférence de 1 % à 3% en poids de ladite composition précurseur ou dudit ingrédient cosmétique.

Plus particulièrement, ladite composition cosmétique peut comprendre :

- 0,5 % à 5 % en poids, de préférence de 1 % à 3% en poids de ladite composition précurseur ou dudit ingrédient cosmétique, et
- de 95 % à 99,5 % en poids d'un ou plusieurs excipients acceptables sur le plan cosmétique.

Dans certains modes de réalisation, la composition cosmétique comprend en outre un ou plusieurs agents actifs à effet cosmétique supplémentaire(s). On entend par « principe actif à effet cosmétique, agent actif à effet cosmétique, agent cosmétique ou actif à effet cosmétique » un composé capable d'exercer au moins un effet cosmétique sur la peau ou ses annexes. On entend par « effet cosmétique » tout effet non thérapeutique visant à modifier et/ou améliorer l'aspect visuel et les propriétés mécaniques de la peau ou des muqueuses comme les lèvres, à les protéger des agressions externes (soleil, vent, humidité, sécheresse, produits chimiques), à prévenir et/ou à corriger les phénomènes liés à leur vieillissement, ou encore à prévenir ou traiter les effets causés par un stress ou la fatigue sur la peau.

Des exemples de tels agents sont, entre autres, les agents antirides, les agents antiâges, les agents antioxydants, les agents hydratants, les agents apaisants, les agents anti-rougeurs, les agents décongestionnants, les agents gommants ou exfoliants, les agents mâtifiants, les agents séborégulateurs, les actifs éclaircissants, les actifs anti-tâches, les agents anticerne ou anti-poche, les antistress, les antifatigues, les actifs anti-pollution, les agents tenseurs, les filtres et écrans solaires, et leurs combinaisons.

Notamment, la composition cosmétique peut comprendre des tocophérols, et/ou des extraits de plantes comme des extraits de graines de lin, des extraits d'exopolysaccharides de *Vibrio,* des peptides comme le trifluoroacétyl tripeptide-2.

Dans un mode particulier, la composition cosmétique peut comprendre un actif choisi parmi un agent antiride, un agent anti-rougeur, un agent antioxydant, un actif hydratant, un agent apaisant, un agent sébo-régulateur, un agent anti-tâche ou éclaircissant, un agent tenseur, un actif anti-pollution, un agent anticerne ou anti-poche, un filtre ou un écran solaire, et leurs combinaisons.

A titre d'exemple d'agents anti-pollution, on peut citer les extraits de *Chrysanthellum Indicum* les polysaccharides, notamment marins issus d'un milieu de fermentation *d'Alteromonas* et les sels de Nigari.

A titre d'exemple d'agents anticerne, on peut citer les extraits de *Chrysantellum Indicum* ou encore les extraits et les polysaccharides sulfatés d'algues, notamment *d'Ascophyllum nodosum* ou *d'Asparagopsis Armata.*

A titre d'exemple d'agents hydratants, on peut citer l'urée, l'acide pidolique (PCA) et ses dérivés en particulier ses sels tels que l'arginine PCA, le chitosan PCA, ses sels de cuivre (Cuivre PCA), de magnésium (magnésium PCA), de sodium (sodium PCA) ou de zinc, l'éthylhéxyl PCA, le gluconate de calcium, l'acide hyaluronique et ses sels et autres glycosaminoglycanes, le frucose, le glucose, l'isomaltose, le lactose, le tréhalose, le polydextrose, le saccharose (Sucrose), le maltitol, le mannitol, le sorbitol, le xylitol et les autres hydrates de carbones et dérivés, les polyéthylène glycols tels que les PEG-7, PEG-8, PEG-10, PEG-12 ou PEG-14, la glycérine, le propylène glycol, le butylène glycol, la betaïne, la citrulline, le collagène et ses dérivés, l'histidine, les hydrolysats de soie, de kératine ou de soja, les extraits de plante riches en polysaccharides et/ou polyphénols, par exemple les extraits d'Aloès, bleuet *(Centaurea cyanus)*, les extraits riches en polysaccharides, notamment issus de milieux de fermentation de microorganismes marins tels que *Alteromonas* et les combinaisons de ceux-ci.

A titre d'exemple d'agents anti-âge, on peut citer l'acide ascorbique et ses dérivés comme l'ascorbyl phosphate de magnésium, les glycosaminoglycanes et leurs dérivés, les polysaccharides de *Cyathea,* le collagène, les extraits de graines de lin (*Linum usitatissimum*), les peptides comme le Caprooyl-Tetrapeptide-3 et le trifluoroacétyl tripeptide-2, les extraits de *Polygonum aviculare,* les extraits d'algues brunes, en particulier *d'Ascophyllum nodosum,* les extraits de fougères, en particulier de *Cyathea cumingii.*

En tant qu'exemple d'agents antistress, on peut citer les produits Rosality™ (combinaison d'une eau de rose et d'une huile essentielle de rose) et l'extrait de ciste à fleurs rose, par exemple commercialisées sous la marque IBR-Chill™.

A titre d'exemple d'agents apaisants, on peut citer l'allantoïne, les extraits d'aloès, de bouleau (par exemple *Betula alba),* d'épilobe (*Epilobium angustifolium),* de châtaignier (par exemple *Castenea sativa*), de bleuet (par exemple *Centaurea cyanus*), de centella (par exemple *Centella asiatica*), de prêle (par exemple *Equisetum arvense*), de fenouil (par exemple *Foeniculum vulgare*), d'hamamélis (par exemple *Hamamelis virginiana*), de lierre (par exemple *Hedera helix*), *d'habiscus sabdariffa,* de lis (par exemple *Lilium candidum*), de mauve (par exemple *Malva sylvestris*), de mélisse (par exemple *Melissa officinalis*), de scutellaire (par exemple *Scutellaria baicalensis*), de mimosa (par exemple *Mimosa tenuiflora*), de potentille (par exemple *Potentilla erecta*), un extrait d'oligosaccharides ou un oligosaccharide, par exemple de lin, les peptides comme le palmitoyl tripeptide-8, les extraits de polysaccharides, notamment les extraits d'exopolysaccharides issus du milieu de fermentation *d'Alteromonas* et les combinaisons de ceux-ci.

A titre d'exemple d'agents antioxydants, on peut citer le HMR (hydroxy méthyl résorcinol), l'acide ascorbique et ses dérivés, la vitamine B9, le chlorhydrate d'histidine, ou un extrait d'épilobe (*Epilobium augustifolium*). Les principes actifs à effet antioxydant et de type vitamine sont généralement utilisés en un pourcentage massique d'au moins 1% par rapport au poids total de la composition cosmétique.

A titre d'exemple d'agents séborégulateurs, on peut citer les lignanes de lin, la poudre de riz, le gluconate de zinc, la sarcosine, un extrait de *Cinnamomum zeylanicum bark,* un extrait d'avocat, un extrait de *Backhousia citriodora* et les combinaisons de ceux-ci.

A titre d'agents anti-rougeurs, on peut citer les saponines, les flavonoïdes, les ruscogénines, les esculosides, et les extraits les contenant, par exemples les extraits de *Ruscus,* ainsi que certaines huiles essentielles, par exemple de lavande ou de romarin.

A titre d'exemple d'agents anti-tâches, on peut citer des extraits comme la réglisse (*Glycyrrhyza glabra*), l'extrait de j ackfruit (*Artocarpus heterophyllus*), les extrait de Rumex (*R.occidentalis),* les extraits de plante appartenant au genre citrus, le resveratrol, les peptides comme l'oligopeptide-68, le nonapeptide-1, l'acide kojique, le magnésium ascorbyl phosphate et les combinaisons de ceux-ci.

Dans un mode de réalisation particulier, ladite composition cosmétique comprend de 0% à 30% en poids, par exemple de 0 à 15% en poids, d'un ou plusieurs agents actifs cosmétiques additionnels.

Dans un mode de réalisation plus particulier, ladite composition cosmétique comprend :

- de 0,0005% à 0,01% en poids, de préférence de 0,001 % à 0,005 % en poids dudit extrait de *Melaleuca alternifolia* selon l'invention,
- de 69,99 % à 99,9995 % en poids d'un ou plusieurs excipients acceptables sur le plan cosmétique, et
- de 0% à 30% en poids, par exemple de 0 à 15% en poids, d'un ou plusieurs agents actifs cosmétiques additionnels.

Dans un mode de réalisation plus particulier, ladite composition cosmétique comprend :

- 0,5 % à 5 % en poids, de préférence de 1 % à 3% en poids de ladite composition précurseur ou dudit ingrédient cosmétique tel que décrit ci-dessus,
- de 65 % à 99,5 % en poids d'un ou plusieurs excipients acceptables sur le plan cosmétique, et
- de 0% à 30% en poids, par exemple de 0 à 15% en poids, d'un ou plusieurs agents actifs cosmétiques additionnels.

L'extrait de *Melaleuca alternifolia* selon l'invention, la composition précurseur ou l'ingrédient cosmétique comprenant, peut être incorporé(e) dans n'importe quel type de composition cosmétique. De préférence, il s'agit d'une composition cosmétique ayant une forme adaptée à l'administration topique, en particulier adaptée à une application sur la peau. La composition cosmétique selon l'invention peut être de tout type. Dans un mode de réalisation, la composition cosmétique est choisie dans le groupe constitué par les solutions aqueuses, les solutions hydroalcooliques, les émulsions huile-dans-eau (H/E) ou eau-dans huile (E/H) ou multiple (triple : E/H/E ou H/E/H), les nanoémulsions, en particulier des nanoémulsions H/E, dont la taille des gouttes est typiquement inférieure à 100 nm, les gels aqueux, ou les dispersions d'une phase grasse dans une phase aqueuse à l'aide de sphérules, les suspensions, de préférence en milieux aqueux ou hydroalcoolique, les suspensions de liposomes, les poudres, les lotions, laits, les crèmes, les onguents, les gels, les mousses, les baumes, et les pommades.

Dans des modes de réalisation, la composition cosmétique selon l'invention est une crème, un baume, un masque, un sérum ou une lotion.

Dans un mode préféré, la composition cosmétique selon l'invention est un sérum, une crème, un masque ou un baume, de préférence de nuit.

## c. Utilisations et Procédés cosmétiques selon l'invention

Selon un aspect supplémentaire, l'Invention a également pour objet l'utilisation de l'extrait de *Melaleuca alternifolia* tel que décrit ci-dessus en tant qu'agent cosmétique régénérateur, réparateur ou anti-fatigue de la peau, et plus particulièrement, pour traiter ou prévenir un ou plusieurs signes de la fatigue cutanée.

L'invention a également pour objet l'utilisation d'une composition cosmétique selon l'invention pour traiter ou prévenir un ou plusieurs signes de la fatigue cutanée.

Au sens de l'invention, les termes « *peau* » et « *cutané* » se réfèrent à toute partie de la peau du corps humain, en particulier la peau du visage, y compris les lèvres et les paupières, le cou, et la peau des mains. De préférence, il s'agit de la peau au niveau du visage, y compris le contour des yeux et le contour des lèvres, et au niveau du cou.

Dans le cadre de la présente invention, l'extrait ou la composition selon l'invention est typiquement appliqué(e) par voie topique, de préférence sur une peau ou une muqueuse. Il s'agit d'une peau ou d'une muqueuse saine, c'est-à-dire une peau ou une muqueuse ne présentant ne présentant pas de plaies ou de pathologies cutanées. En particulier, l'extrait ou la composition selon l'invention est appliqué(e) sur une peau non-acnéique (c'est-à-dire ne souffrant pas d'acné), ne présentant pas de plaies, notamment résultant de piqures d'insectes, ou d'infections cutanées, e.g. causées par un champignon (mycose), une bactérie ou un protozoaire.

En d'autres termes, l'extrait ou la composition selon l'invention ne produit pas d'effet thérapeutique sur la peau, lorsqu'elle/il est utilisé(e) selon l'invention.

On entend par « *agent anti-fatigue* » un agent cosmétique capable de prévenir, de traiter ou d'atténuer les « *effets ou signes cutanés* » de la fatigue, c'est-à-dire les effets de la fatigue sur la peau.

Dans le cadre de la présente invention, on entend par « *signes ou effets cutanés* », toute altération ou modification non-pathologique de l'aspect visuel ou des propriétés mécaniques de la peau. Dans le cadre de la présente invention, les « *signes ou effets cutanés* » sont causés par, ou associés à, la fatigue.

On entend par *« prévenir un signe ou un effet »,* le fait de retarder ou d'empêcher l'apparition dudit signe ou effet sur la peau. On entend par « *traiter un signe ou un effet »,* le fait de diminuer, d'atténuer, d'estomper, de corriger ou ralentir le développement dudit signe ou effet sur la peau. Le ou les signes de la fatigue cutanée peuvent par exemple être une perte d'éclat de la peau, un teint terne, un teint brouillé, une perte d'uniformité du teint, les traits du visage tirés, des cernes au niveau des yeux, des poches au niveau des yeux, les paupières gonflées, une altération de l'aspect lisse de la peau, une augmentation de la rugosité de la peau, une perte d'élasticité, une perte de densité, une perte de fermeté, une perte d'hydratation, une apparition de ridules ou de rides, une apparition de rougeurs, et leurs combinaisons.

Dans le cadre de la présente invention, la fatigue causant les modifications ou altérations non pathologiques de la peau peut être de tout type. Il peut s'agir d'une fatigue passagère ou chronique. Il s'agit notamment d'une fatigue causée par un ou plusieurs de facteurs notamment environnementaux. Il peut d'agir de facteurs tels que le manque de sommeil par exemple dû au rythme de travail ou à la situation familiale (e.g. naissance d'un enfant), des troubles de sommeil ou le décalage horaire (jet-lag), une hygiène alimentaire mauvaise ou déséquilibrée, la consommation d'alcool, le manque ou l'excès d'activité physique, la sédentarité, le surmenage, une activité intense ou inadaptée au travail, une activité de nuit ou en horaire décalé, une exposition importante aux écrans, le stress, l'anxiété ou des angoisses liées à la vie professionnelle ou familiale.

La fatigue peut résulter également de situations physiologiques telles que la grossesse ou la ménopause.

De préférence, la fatigue n'est pas associée ou causée par un état pathologique chez l'individu. Dans certains modes de réalisations, l'extrait de *Melaleuca alternifolia* selon l'invention peut être utilisé, pour au moins une (1, 2, 3, 4, 5, 6, 7, 8, 9 ou 10) des finalités suivantes :

- Prévenir, traiter ou atténuer les cernes et/ou les poches aux niveaux des yeux,
- Prévenir, traiter ou atténuer le gonflement des paupières
- Prévenir, traiter ou réduire les irrégularités de pigmentation de la peau, y compris les tâches pigmentaires induites par la fatigue,
- Homogénéiser ou unifier le teint du visage,

- Rendre le teint du visage plus lumineux et/ou plus éclatant et/ou plus clair,
- Prévenir ou traiter un teint terne, un teint brouillé et/ou des traits tirés,
- Rendre la peau, notamment au niveau du visage, plus fraîche et plus lumineuse,
- Apaiser les traits du visage,
- Rendre l'aspect de la peau moins fatigué, plus relaxé, plus frais
- Rendre le regard moins fatigué, plus reposé et plus lumineux,
- Promouvoir ou accélérer la récupération de l'épiderme, notamment au niveau du visage,
- Revitaliser la peau, notamment au niveau du visage,
- Promouvoir un effet bonne mine,
- Prévenir, traiter l'altération de, ou restaurer, la fonction barrière de la peau,
- Prévenir, traiter, ou atténuer, une perte de fermeté de la peau, notamment au niveau du visage,
- Prévenir, traiter ou atténuer, la déshydratation de la peau (ou de manière équivalente « perte d'hydratation »),
- Prévenir, traiter, retarder, ou atténuer, l'apparition de rides ou de ridules, et/ou
- Prévenir traiter ou atténuer, l'apparition de rougeurs.

Il va sans dire que les altérations non pathologiques ci-dessus listées sont associées ou causées par la fatigue sur la peau.

Dans certains modes de réalisation, l'extrait selon l'invention peut être utilisé en tant qu'agent cosmétique pour prévenir, traiter ou atténuer un ou plusieurs signes de la fatigue cutanée choisis parmi une déshydratation de la peau, une perte d'élasticité, une perte de fermeté, des rides, des cernes et les combinaisons de ceux-ci.

Dans le cadre de la présente invention, on entend par « *agent régénérateur* ou *réparateur* » un agent cosmétique capable de promouvoir ou stimuler la régénération de la peau ou la réparation de la peau pendant le sommeil de telle sorte que les effets cutanés causés par la fatigue sont atténués, traités ou prévenus. Il peut s'agir notamment d'une fatigue chronique ou causée par un ou plusieurs facteurs notamment environnementaux. A cet égard, la Demanderesse a notamment observé que l'extrait selon l'invention stimule les gènes impliqués dans la synthèse du collagène au niveau de la peau.

Comme cela est illustré dans les exemples, l'application de l'extrait sur la peau à une très faible concentration, notamment non-perceptible par l'odorat, permet d'améliorer le sommeil.

Il a été également montré que l'extrait selon l'invention est capable d'activer les voies de signalisation de la mélatonine, notamment celles impliquées dans les mécanismes de réparation de l'ADN, et de défense cellulaire contre le stress oxydant mais également dans la synthèse et le transport de la mélatonine, par exemple induite par une exposition à un stress environnemental telle qu'une exposition excessive aux UV

Ainsi, dans un mode de réalisation particulier, l'extrait selon l'invention est utilisé en tant qu'agent pour activer la voie de signalisation de la mélatonine et/ou potentialiser l'effet de la mélatonine au niveau de la peau.

Selon un aspect particulier, l'extrait selon l'invention est utilisé pour favoriser ou améliorer la récupération cutanée, notamment pendant le sommeil. L'extrait selon l'invention est notamment utilisé pour potentialiser les mécanismes naturels de réparation et de régénération de la peau contrôlés par la mélatonine.

Dans un aspect supplémentaire ou alternatif, l'extrait selon l'invention est utilisé pour améliorer le sommeil, notamment pour augmenter la durée totale du sommeil et/ou celle du sommeil profond.

Selon un mode de réalisation supplémentaire, l'extrait selon l'invention est utilisé pour promouvoir ou favoriser la récupération cutanée pendant la phase de sommeil.

Selon un aspect supplémentaire, l'extrait selon l'invention est utilisé pour réparer/régénérer la peau chez un individu exposé à une fatigue, notamment à une fatigue chronique.

L'extrait selon l'invention peut être utilisé pour réparer/régénérer la peau exposée à un stress, notamment un stress environnemental tel qu'une exposition au soleil, au vent, à l'humidité, à la sécheresse ou à des produits chimiques ou des procédés de type abrasion, rayons etc... ou encore à un stress lié au mode de vie (décalage horaire, manque de sommeil, travail de nuit etc...).

De manière additionnelle ou alternatif, l'extrait selon l'invention est utilisé pour induire la synthèse de collagène au niveau de la peau.

De manière additionnelle ou alternatif, l'extrait selon l'invention peut être également utilisé en tant qu'agent pour réduire la formation d'espèces réactives de l'oxygène (ROS) et/ou pour stimuler l'expression de protéines antioxydantes, notamment d'enzymes antioxydantes au niveau de la peau. Dans un mode de réalisation particulier, l'extrait *de Melaleuca alternifolia* selon l'invention est présent en tant qu'agent actif cosmétique dans une composition cosmétique, de préférence une crème, un baume, un masque, un sérum ou une lotion, comme cela est décrit ci-avant. La concentration de l'extrait *Melaleuca alternifolia* selon l'invention dans une telle composition cosmétique est avantageusement faible, de sorte qu'elle n'est pas perceptible par l'odorat. Typiquement, cette concentration est de 0,0005% à 0,01% en poids, de préférence de 0,001 % à 0,005 % en poids dudit extrait.

Il va de soi que de telles utilisations sont destinées à des individus exposés à la fatigue telle que définis ci-avant.

Les individus visés par la présente invention peuvent être de tout âge et genre. Dans un mode de réalisation préféré, il s'agit d'un individu ayant moins de 65 ans. Il peut s'agir, par exemple, d'une femme ou d'un homme de 25 à 65 ans, notamment de 25 à 55 ans. A titre d'exemple, il s'agit d'une femme âgée de 25 à 45 ans.

Un objet supplémentaire de la présente invention est un procédé cosmétique pour prévenir ou traiter les signes de la fatigue cutanée chez un individu, ledit procédé comprenant l'application d'une quantité cosmétiquement efficace d'un extrait de *Melaleuca alternifolia* selon l'invention, ou d'une composition cosmétique selon l'invention sur la peau.

Le procédé cosmétique selon l'invention a pour but de traiter ou prévenir un ou plusieurs effets cutanés de la fatigue, de préférence choisis parmi une altération du teint de la peau, notamment un teint brouillé, un teint terne, une perte d'éclat du teint ou une perte d'uniformité du teint, notamment l'apparition de tâches pigmentaires, des traits du visage tirés, l'apparition de poches et/ou de cernes au niveau des yeux, des paupières gonflées, une altération de l'aspect lisse de la peau, une augmentation de la rugosité de la peau, une perte d'élasticité, une perte de densité, une perte de fermeté, une perte d'hydratation, une apparition de rides, une apparition de rougeurs, et leurs combinaisons.

Le procédé selon l'invention peut permettre d'obtenir l'un quelconque des effets suivants, chez un individu exposé à la fatigue :

- Prévenir, traiter ou atténuer les cernes et/ou les poches aux niveaux des yeux,
- Prévenir, traiter ou atténuer le gonflement des paupières
- Prévenir, traiter ou réduire les irrégularités de pigmentation de la peau, y compris les tâches pigmentaires induites par la fatigue,
- Homogénéiser ou unifier le teint du visage,
- Rendre le teint du visage plus lumineux et/ou plus éclatant et/ou plus clair,
- Prévenir ou traiter un teint terne, un teint brouillé et/ou des traits tirés,
- Rendre la peau, notamment au niveau du visage, plus fraîche et plus lumineuse,
- Apaiser les traits du visage,
- Rendre l'aspect de la peau moins fatigué, plus relaxé, plus frais
- Rendre le regard moins fatigué, plus reposé et plus lumineux,
- Promouvoir ou accélérer la récupération de l'épiderme, notamment au niveau du visage,
- Revitaliser la peau, notamment au niveau du visage,
- Promouvoir un effet bonne mine,
- Prévenir, traiter l'altération de, ou restaurer, la fonction barrière de la peau,
- Prévenir, traiter, ou atténuer, une perte de fermeté de la peau, notamment au niveau du visage,
- Prévenir, traiter ou atténuer, la déshydratation de la peau (ou de manière équivalente « perte d'hydratation »),
- Prévenir, traiter, retarder, ou atténuer, l'apparition de rides, et/ou
- Prévenir traiter ou atténuer, l'apparition de rougeurs.

Dans les méthodes et utilisations cosmétiques selon l'invention, la dose à administrer et la fréquence d'administration de la combinaison selon l'invention varient en fonction de l'effet cosmétique recherché, des caractéristiques de l'individu, en particulier de son sexe, de son âge et de son type de peau. Typiquement, l'extrait de *Melaleuca alternifolia* selon l'invention, ou la composition cosmétique la comprenant, peut être appliquée sur la zone à traiter une fois par jour, de préférence avant le coucher, pendant plusieurs semaines consécutives voire plusieurs mois, par exemple au moins pendant 3 mois. A titre d'exemple, le patient peut appliquer une dose de 1 g à 2 g de composition cosmétique sur son visage le soir.

Définitions générales:

Sauf indication contraire, tous les termes techniques et scientifiques utilisés ici ont la même signification que celle couramment comprise par l'homme du métier du domaine technique de la présente demande.

Cette demande n'est pas limitée par les exemples de procédés, utilisations et compositions décrits ici, et tous les procédés, utilisations et compositions similaires ou équivalents à ceux décrits ici peuvent être utilisés dans les modes de réalisation de la présente demande.

Les en-têtes et titres de section fournis ici ne doivent pas être considérés comme des limitations des divers aspects ou modes de réalisation de la présente demande. Les en-têtes et titres de section sont utilisés ici à des fins d'organisation uniquement et ne doivent pas être interprétés comme limitant l'invention décrite. Tous les termes définis sont plus complètement définis par référence à la demande dans son ensemble.

Toutes les publications, y compris les documents de brevet et les articles scientifique, mentionnés dans cette demande sont incorporés par référence dans leur intégralité dans la même mesure que si chaque publication individuelle était incorporée individuellement par référence. La citation de ces publications ne doit en aucun cas être interprétée comme une admission que de telles publications constituent un art antérieur. Si une définition énoncée dans la présente demande est contraire ou incompatible avec une définition énoncée dans les brevets, demandes, demandes publiées et autres publications qui sont incorporées ici par référence, la définition énoncée dans la présente demande prévaut sur la définition qui est incorporée par référence. Toutes les caractéristiques décrites dans cette demande peuvent être combinées dans n'importe quelle combinaison. Chaque caractéristique décrite dans la présente demande peut être remplacée par une autre caractéristique ayant le même objectif, un objectif équivalent ou similaire. Ainsi, sauf indication expresse contraire, chaque caractéristique divulguée n'est qu'un exemple d'une série générique de caractéristiques équivalentes ou similaires.

Des définitions de termes peuvent apparaître tout au long de la description. Il doit être compris que cette description n'est pas limitée aux modes de réalisation particuliers décrits, car ceux-ci peuvent, bien entendu, varier. Il faut également comprendre que la terminologie utilisée ici a pour seul but de décrire des modes de réalisation particuliers et n'est pas destinée à être limitative.

Il convient de noter que, telles qu'elles sont utilisées ici et dans les revendications annexées, les formes singulières « un », « une » et « le » incluent des référents pluriels à moins que le contexte n'indique clairement le contraire. Par exemple, "un" ou "une" incluent "au moins un" et "un ou plusieurs".

Les termes « comprenant », « comprend » et « composé de » tels qu'utilisés ici sont synonymes de « incluant », « inclut », « contenant », « contient », et leurs variantes grammaticales. Ces termes sont inclusifs ou ouverts et n'excluent pas que des membres, des éléments ou des étapes de procédé supplémentaires non cités soient présents

Lorsqu'une plage de valeurs est prévue, il est entendu que chaque valeur intermédiaire entre les bornes supérieure et inférieure de cette plage est également spécifiquement divulguée et englobée dans cette description.

## EXEMPLES

L'invention sera mieux comprise à la lumière des exemples suivants, qui sont donnés à titre purement illustratif et n'ont pas pour but de limiter la portée de l'invention.

### EXEMPLE 1 : Préparation d'un extrait selon l'invention

#### 1. Obtention d'un extrait de *Melaleuca alternifolia* selon l'invention

Comme indiqué dans la description, l'extrait selon l'invention peut être obtenu par distillation fractionnée d'une huile essentielle standard de Melaleuca alternifolia. L'huile essentielle a été obtenue à partir de feuilles et de branches terminales fraîches par entraînement à la vapeur. L'huile essentielle a été ensuite fractionnée par distillation. Une première fraction riche en gamma-terpinène a été obtenue, puis une deuxième fraction riche à la fois en gamma-terpinène et terpinén-4-ol, suivie d'une troisième fraction et d'une quatrième fraction, toutes les deux riches en terpinén-4-ol. Enfin, l'extrait selon l'invention, correspondant à la cinquième fraction, a été obtenu.

Le tableau 1 détaille les caractéristiques physiques et chimiques de l'huile essentielle de *Melaleuca alternifolia* distillée à la vapeur avant la distillation fractionnée. Le tableau 2 détaille la composition du matériau de départ (« première huile essentielle ») et celle de la fraction d'intérêt (« huile essentielle selon l'invention »).

Les analyses GC/FID ont été réalisées avec un appareil Agilent 6890 (Agilent Technologies) équipé d'un détecteur à ionisation de flamme (FID), d'un échantillonneur automatique de liquide et de la même colonne capillaire HP-1MS (100% diméthylpolysiloxane, 60 m x 0,15 mm i.d., épaisseur du film 0,25 $\mu$m ; Agilent Technologies).. La température du four a été maintenue à 60°C pendant 10 min, puis programmée de 60 à 300°C avec 2°C/min, et enfin 300°C pendant 10 min (temps de passage 140 min). Les températures de l'injecteur et du détecteur étaient respectivement de 250°C et 300°C ; $H_2$ (1.0 ml/min) comme gaz porteur ; injection en mode split (1:50) ; volume injecté 0.1 ml. Les débits d'$O_2$ d'$H_2$ et de gaz d'appoint du FID étaient de 350, 35 et 20 ml/min, respectivement.

Tableau 1 : Caractéristiques de l'huile essentielle de départ

| CARACTERISTIQUES PHYSICO-CHIMIQUES DE L'HUILE ESSENTIELLE DE DEPART | |
|---|---|
| APPARENCE | LIQUIDE |
| COULEUR | INCOLORE - JAUNE CLAIR |
| ODEUR | CARACTERISTIQUE |
| DENSITÉ RELATIVE (20°C/20°C) | 0,885 - 0,906 |
| INDICE DE RÉFRACTION (20°C) | 1,475 - 1,482 |
| POUVOIR ROTATOIRE (20°C) | +5° A + 15° |
| HUMIDITE (VISUELLE) | PAS D'EAU VISIBLE A 20°C |

(suite)

| CARACTERISTIQUES PHYSICO-CHIMIQUES DE L'HUILE ESSENTIELLE DE DEPART | |
|---|---|
| MISCIBILITE DANS L'ETHANOL 85% (v/v) A 20°C | 1 VOLUME D'HUILE DONNE UNE SOLUTION CLAIRE DANS AU PLUS 2 VOLUME D'ETHANOL 85% (v/v) |
| POINT FLASH | 50°C - 60°C |

Tableau 2: composition en composés volatils de l'huile essentielle de départ et de l'Extrait selon l'Invention.

| COMPOSES | TENEUR % (AIRE GC-FID) | |
|---|---|---|
| | HUILE ESSENTIELLE DE DEPART | EXTRAIT SELON L'INVENTION |
| alpha-Pinène | 1,0 - 6,0 | - |
| Sabinène | JUSQU'À 3,5 | - |
| alpha-Terpinène | 5,0 - 13,0 | 0,02 |
| Limonène | 0,5 - 1,5 | - |
| para-Cymène | 0,5 - 4,0 | 0,22 |
| 1,8-Cinéole | JUSQU'À 5,0 | - |
| gamma-Terpinène | 10,0 - 28,0 | 0,01 |
| alpha-Terpinolène | 1,5 - 5,0 | 0,01 |
| Terpinèn-4-ol | 37,0 - 45,0 | 0,16 |
| alpha-Terpinéol | 1,5 - 8,0 | 1,09 |
| Aromadendrène | JUSQU'À 3,0 | 8,65 |
| Ledène | JUSQU'À 3,0 | 17,80 |
| delta-Cadinène | JUSQU'À 3,0 | 17,29 |
| Globulol | JUSQU'À 1,0 | 3,25 |
| Viridifloral | JUSQU'À 1,0 | 2,61 |

**EXEMPLE 2** : **Evaluation de l'extrait selon l'invention sur des explants de peau**

Protocole : Le but de cette étude était d'évaluer l'effet réparateur de l'extrait selon l'invention sur des explants de peau et de démontrer son activité « mélatonine-like » sur la peau. L'extrait selon l'invention obtenu à l'Exemple 1 a été dilué dans du pentylène glycol à une concentration de 0,5% en poids. Cette composition précurseur (également appelé ingrédient cosmétique) a été ensuite formulée dans du carbopol à une concentration de 1% en poids. C'est cette composition finale qui a été utilisée dans cette expérience.

Sur une plastie abdominale provenant d'une femme caucasienne de 46 ans (référence : P2471-AB46, de phototype II-III (selon la classification Fitzpatrick de la couleur de peau), 51 explants d'un diamètre moyen de 11 mm (+ 1 mm) ont été préparés. Les explants ont été maintenus en vie dans un milieu BEM (BIO-EC's Explants Medium) à 37°C en atmosphère humide à 5% de $CO_2$ pendant 2 jours. Au 2ème jour, le milieu de culture a été remplacé par une solution de HBSS puis les explants ont été irradiés par des UV (groupe « UV-Placebo » et groupe « UV-Extrait) avec une dose de 13,5 J/cm2 d'UVA correspondant à 3 MED (dose érythémale minimale) et une dose de 0,15 J/cm2 d'UVB correspondant à 1 MED ou non (groupe « Placebo»). A la fin de l'irradiation, le produit placebo (pour les groupe « Placebo » et groupe « UV-Placebo ») ou la composition comprenant l'extrait selon l'Invention (« UV-extrait») ont été appliqués par voie topique sur la surface cutanée des explants correspondants à raison de 2µL par cm² explant (≈ 2mg/cm²) pour 24h supplémentaire. A J3, les explants ont été fixés dans du RNAlater avant extraction de l'ARN en utilisant ReliaPrep™ RNA Tissue Miniprep System (fibrous) de chez Promega. La qualité et la concentration des ARNs ont été évaluées avant d'effectuer l'étape de transcriptase inverse avec iScript (Bio-Rad, 20µl/réaction). Avec l'aide d'un contrôle spike, le ∆Cq a été calculé. Les benchmarks sont évalués pour chaque réaction qPCR à l'aide du logiciel CFX Manager 3.1 sont les suivants :

- La courbe logarithmique de l'amplification pour chaque séquence d'intérêt.
- Les courbes de fusion sont des intensités de fluorescence enregistrées après le dernier cycle. La température passe de 65° à 96°C avec un incrément de 0,5°C toutes les 5 secondes.

- Les courbes représentant l'inverse de la dérivée du signal de fluorescence en fonction de la température (en °C), permettent de vérifier un seul amplicon.

Tous les échantillons ont été soumis à une analyse du profil d'expression génique. Pour la quantification, le nombre de cycle a été normalisé par rapport au gène de référence B2M :

(i)

$$Cq \text{ (quantité relative) par échantillon et par gène} = E \text{ (gène) (Cq (contrôle) - Cq (traité))}$$

$$E = \text{efficacité des paires d'amorces} = (\% \text{ efficacité}*0{,}01) + 1$$

Cq (contrôle) = le seuil quantitatif calculé par le CFX Maestro en mode "régression" pour la condition de contrôle.
Cq (traité) = le seuil quantitatif calculé par le CFX Maestro en mode "régression" pour la condition traitée.
gene = gène cible

(ii) Expression d'un gène cible normalisé = NE NE=Expression of normalized sample (gene) = Cq sample (gene) / ((Cq sample (ref 1) * Cq sample (ref 2))$^{1/n}$

Cq = quantité relative
le dénominateur correspond au facteur de normalisation incluant les deux gènes de référence : ref 1 = B2M
gène =gène cible

Pour chaque gène d'intérêt, les valeurs ont été calculées entre les échantillons de traitement en triplicata par rapport aux échantillons témoins d'explants appariés en triplicata pour chaque point de temps cinétique. Un gène a été considéré comme induit si son expression a montré une augmentation supérieure ou égale à 1,5 par rapport au contrôle (fold-change $\geq 1{,}5$). De même, un gène a été considéré comme réprimé s'il présentait une réduction de son expression par rapport au contrôle (fold-change $\leq 0{,}65$). De plus, lorsqu'une modulation génique homogène est conservée entre les triplicats biologiques, une valeur P <0,05 (selon le test T) est indiquée par un astérisque.

Résultats : Par rapport à la condition UV avec placebo (UV-placebo), l'extrait a déclenché une forte induction d'OGG1 qui est impliqué dans la réparation de l'ADN. De plus, l'extrait a induit une forte induction d'enzymes antioxydantes telles que CAT et GPX1. Un effet sur COL3A1 et COL1A2 tend également à prouver que l'extrait selon l'invention agit sur la matrice extracellulaire avec une augmentation de la synthèse de collagène. Fait intéressant, l'extrait a induit une augmentation de l'expression des gènes associés à la mélatonine (ASMTL, ROR$\alpha$, SIRT3 et SLC15A1) ce qui tend à prouver que l'extrait cible la même voie que la mélatonine et est donc capable d'activer les voies de signalisation de la mélatonine, notamment celles impliquées dans les mécanismes protection contre le stress oxydant, notamment induit par les UV. La stimulation de cette voie de signalisation expliquerait la stimulation ou la répression des gènes mentionner ci-dessus.

L'ensemble de ces résultats supportent l'utilisation de l'extrait selon l'invention en tant qu'agent antifatigue, régénérateur ou réparateur de la peau.

Tableau 3 : Résultats d'expression génique

|  | UV-Extrait vs UV-placebo | P-Value |
|---|---|---|
| ASMTL | 1,55 | 0,042 |
| ROR$\alpha$ | 1,63 | 0,040 |
| SIRT3 | 1,62 | 0,008 |
| SLC15A1 | 2,62 | 0,007 |
| CAT | 1,94 | 0,041 |
| GPX1 | 1,70 | 0,033 |

(suite)

|  | UV-Extrait vs UV-placebo | P-Value |
|---|---|---|
| COL3A1 | 2,56 | 0,013 |
| COL1A2 | 1,97 | 0,012 |
| OGG1 | 2,02 | 0,005 |

**EXEMPLE 3 : Amélioration du sommeil**

Protocole : 32 participants sains (16 hommes, 22-56 ans, âge médian 41,5 ans) avec des troubles du sommeil légers auto-déclarés, causés par des facteurs liés au mode de vie, mais sans diagnostic de trouble du sommeil clinique ont été sélectionnés

L'étude a consisté en l'évaluation de 2 échantillons de crème pour le visage, 1 crème « actif » et une « crème placebo», utilisés séparément sur 3 semaines :

- Produit A-crème « actif » : crème générique non parfumée pour le visage comprenant 1% d'une composition précurseur comprenant 0,25 % en poids de l'extrait de l'Exemple 1, dans du pentylène glycol (A-Leen® 5, Minasolve),
- Crème « Placebo » : crème générique (cf. exemple 5, tableau 5 ci-après) non parfumée pour le visage comprenant 1% de pentylène Glycol (A-Leen® 5, Minasolve) seul.

Appareils : pour des mesures objectives du sommeil, les participants ont utilisé l'appareil SleepScore Max (SleepScore Labs, Carlsbad, CA), un appareil de surveillance sans contact qui utilise le signal respiratoire et la détection de mouvement pour détecter le sommeil et qui a été validé par la polysomnographie (PSG), méthode standard de mesure du sommeil. Toutes les données d'auto-déclaration ont été recueillies à l'aide de Compusense, un logiciel de collecte de données en ligne.

Conception et procédure de l'étude :

- Etude en contexte, randomisée et contrebalancée utilisant une crème contrôle non parfumée
- Les participants ont appliqué les crèmes au moment du coucher, dans leur environnement de sommeil naturel.
- L'étude a duré du dimanche soir au vendredi matin pendant 3 semaines consécutives.
- Les participants ont utilisé un échantillon différent chaque semaine. L'échantillon contrôle a été utilisé au cours de la deuxième semaine. Le tableau 3 montre un aperçu de la conception de l'étude.
- Pour chaque nuit de l'étude, les participants ont rempli des questionnaires quotidiens et suivi leur sommeil à l'aide de l'appareil SleepScore Max.

Tableau 4 : détails de l'essai clinique

|  | Participants | Echantillon testé |
|---|---|---|
| Semaine 1 | Groupe (N=16) | Extrait selon l'invention |
| Semaine 2 | Groupe (N=32) | Placebo |
| Semaine 3 | Groupe (N=16) | Extrait selon l'invention |

Analyses de données :

Les données objectives et autodéclarées sur le sommeil nocturne ont été analysées à l'aide du logiciel statistique JMP Pro (version 15) utilisant un modèle mixte, chez les participants. Un niveau alpha de 0,05 a été utilisé pour tous les tests statistiques. La comparaison a été faite entre le contrôle et l'extrait. Les pourcentages proviennent de calcul par rapport à la condition contrôle.

Méthodes statistiques :

Les données obtenues et les variations en pourcentage ont été soumises au test bidirectionnel t de Student pour les données appariées. La valeur de signification statistique est $p < 0,05$.

Résultats :

L'extrait selon l'invention a augmenté de manière significative la durée totale du sommeil ($p < 0,05$) par rapport au contrôle, en particulier la durée du sommeil profond ($p < 0,05$). Les participants ont en moyenne dormi pendant 6,5 heures (392 minutes) par nuit avec la crème contrôle non parfumée contre 6,7 heures (402 minutes) avec la crème contenant l'extrait selon l'invention. La durée du sommeil profond est également passée de 1,2 heure (73 minutes) avec la crème contrôle à 1,3 heure (79 minutes) avec la crème contenant l'extrait selon l'invention ($p<0,05$).

**EXEMPLE 5** : **Produits cosmétiques intégrant l'extrait selon l'invention**

A) Crème de nuit comprenant 1% d'une composition précurseur (également appelé ingrédient cosmétique) comprenant 0,25% en poids de l'extrait selon l'invention préparé selon l'Example 1 dans le pentylène glycol

Tableau 5 : Composition d'une crème de nuit

| Nom commercial | INCI | % en poids |
|---|---|---|
| EAU DEIONISEE | AQUA (eau) | 71,65 |
| CARBOPOL ULTREZ 20 | ACRYLATES/C10-30 ALKYL ACRYLATE CROSSPOLYMERE | 0,30 |
| ARLACEL 165 | GLYCERYL STEARATE (et) PEG-100 STEARATE | 6,00 |
| DUB ININ | ISONONYL ISONONANOATE | 15,00 |
| LIPEX SHEA™ | Beure de Karité (BUTYROSPERMUM PARKII (SHEA) BUTTER) | 5,00 |
| DEKABEN C4 | PHENOXYETHANOL (et) METHYLPARABENE (et) ETHYLPARABENE (et) BUTYLPARABENE (et) PROPYLPARABENE | 1,00 |
| SODIUM HYDROXIDE | SODIUM HYDROXIDE | 0,05 |
| « Extrait » dans A-Leen-5® | Pentylène glycol et huile d'arbre à thé | 1,00 |
| | | **100,00** |

B) Crème anti-fatigue comprenant 1% une composition précurseur comprenant 0,25% en poids de l'extrait selon l'invention préparé selon l'Example 1 dans la triheptanoïne.

[Table 6]

Tableau 6 : Composition d'une crème anti-fatigue

| Nom Commercial | INCI | % en poids |
|---|---|---|
| DEAU DEIONISEE | AQUA (eau) | 79,30 |
| SATIAXANE™ VPC 911 | GOMME de XANTHANE | 0,50 |
| DERMOFEEL® GSC | GLYCERYL STEARATE CITRATE | 2,00 |
| DERMOFEEL® PS | POLYGLYCERYL-3 STEARATE | 2,00 |
| HUILE D'AMANDE DOUCE | HUILE DE PRUNUS AMYGDALUS DULCIS | 3,00 |
| LANETTE® 16 | CETYL ALCOOL | 2,00 |
| LIPEX SHEASOFT™ | BEURRE de BUTYROSPERMUM PARKII | 3,00 |
| CETIOL® C5 | COCO-CAPRYLATE | 3,00 |

(suite)

| Nom Commercial | INCI | % en poids |
|---|---|---|
| VITAPHEROLE® E-1000 | TOCOPHEROL (et) HUILE DE GRAINES D'HELIANTHUS ANNUUS (tournesol) | 0,20 |
| DERMOSOFT® 1388 | GLYCERINE (et) SODIUM ANISATE (et) SODIUM LEVULINATE (et) AQUA (eau) | 4,00 |
| « Extrait » dans Triheptanoine | Triheptanoïne et huile d'arbre à thé | 1,00 |
| | | **100,00** |

**EXAMPLE 6 : Amélioration des signes de fatigue de la peau**

Protocole : Un essai clinique a été réalisé afin de confirmer l'effet antifatigue de l'extrait selon l'Invention. Quarante-quatre volontaires ont été répartis uniformément en deux groupes (22 individus dans le groupe test : Crème « actif » et 22 individus dans le groupe placébo : crème Placébo).

Les volontaires enrôlés étaient des personnes travaillant en horaire décalé, des jeunes adultes très actifs socialement et ayant l'habitude de sortir et de se coucher tard ou de voyager (jet-lag), ou des jeunes mamans. Les volontaires avaient donc des cycles de sommeil perturbés mais n'étaient pas traités pour des troubles du sommeil.

L'étude a été réalisée pendant 28 jours avec des mesures à J0, T12h, J7 et J28. Les produits ont été appliqués une fois par jour par les volontaires, le soir avoir avant d'aller se coucher.

Les 2 crèmes testées sont :

- Crème « actif » : crème générique non parfumée pour le visage comprenant 1% d'un ingrédient cosmétique comprenant 0,25 % en poids de l'extrait de l'Exemple 1, dans de la Triheptanoïne (cf. exemple 5, tableau 5 ci-avant),,
- Crème « Placebo » : crème générique (cf. exemple 5, tableau 5 ci-avant sans l'extrait) non parfumée pour le visage comprenant 1% de la Triheptanoïne seule.

Appareils : pour observer les signes de fatigue, les paramètres suivants ont été mesurés :

- La perte insensible en eau (PIE), évaluée au moyen de la mesure Tewameter® TM 300 (Courage+Khazaka, electronic GmbH).
- L'hydratation de la peau, évaluée avec un Corneometer®
- Le potentiel antioxydant de la peau, mesuré à partir d'échantillons des premières couches du stratum corneum prélevés à J0 et J28 sur les joues par l'expérimentateur à l'aide de Corneofix® (Courage+Khazaka,electronic GmbH). Le test FRAP (Ferric Reducing Antioxidant Parameter) est ensuite réalisé.
- Les rides au niveau de la patte d'oie, mesurée avec le Primos 3D (GFMesstechnik GmbH).
- L'élasticité et la fermeté de la peau, mesurées par la méthode d'aspiration/élongation (Cutometer®MPA 580, Courage+Khazaka, electronic GmbH). Cet appareil applique une pression négative (aspiration) où la peau est aspirée dans la cupule de mesure de la sonde qui permet de mesurer sa profondeur de pénétration par un système de mesure optique.
- L'évaluation des cernes, à partir d'images originales prises avec le scanner à lumière bleue Bio pour chaque volontaire et traitées par un logiciel spécifique, a été mesurée à l'aide de l'angle de typologie individuelle (ITA°).

Méthode statistique :

Pour chaque temps, une étude statistique a été réalisée pour analyser la significativité de l'évolution des paramètres. Les données obtenues et les variations en pourcentage ont été soumises à une A-NOVA bidirectionnel. La valeur de signification statistique est $p<0,05$ ($*p<0,05$ ; $**p<0,01$ ; $***p<0,001$ ; $****p<0.0001$).

Résultats :

Les résultats de cet essai clinique sont illustrés par les Figures 4 à 10.

Le groupe traité avec la crème « actif » présente une diminution significative, de la perte insensible en eau (PIE) de la peau , qui augmente dans le temps, par rapport au groupe traité avec la crème « placebo ». Ceci souligne une amélioration de la fonction barrière (Figure 4). Une amélioration significative de l'hydratation de la peau a été également observée dans le groupe traité avec la crème « actif » avec une augmentation de +7.5%**, +7.9%** et +12%**** après 12h, 7J et 28J, respectivement (Figure 5).

Une augmentation significative du potentiel antioxydant de la peau a été également observée dans le groupe traité avec la crème «actif » en comparaison avec le groupe traité avec la crème placebo (réduction de $Fe^{3+}$ en $Fe^{2+}$ de +17% à 28J par rapport au groupe placebo, Figure 6). Une amélioration de l'aspect lisse de la peau et de ses propriétés biomécaniques a été observé dans le groupe traité avec la crème « actif » en comparaison avec le groupe « placebo ». Ainsi, la crème « actif » comprenant l'extrait selon l'Invention a permis de lisser la peau et réduire rapidement l'apparence des rides, comme l'atteste la diminution du paramètre Sa de -6,9%*** et -9%**** après 7J et 28J (Figure 7).

Une diminution nette des rides au niveau des pattes d'oie également observable visuellement sur les clichés pris à 0J et à 28J et également confirmé par les mesures réalisées par le Primos 3D.

La crème « actif » a également amélioré les propriétés biomécaniques de la peau en augmentant sa fermeté et son élasticité comme cela est illustré par une diminution significative du paramètre R0 (Figure 8) et une augmentation significative du paramètre R2 (Figure 9) par rapport au groupe placebo.

Enfin, la crème « actif » a induit un éclaircissement rapide et significatif des cernes sous les yeux par rapport au groupe placebo (Figure 10).

En conclusion, cet essai clinique a montré que l'extrait de *Melaleuca alternifolia* est un puissant agent antifatigue de la peau. L'application journalière d'une crème comprenant 0,0025% de l'extrait selon l'invention a permis d'améliorer les signes de la fatigue cutanée chez des personnes présentant des cycles de sommeil perturbés.

L'Extrait selon l'Invention a notamment permis d'améliorer la fonction barrière de la peau, d'améliorer le pouvoir antioxydant de la peau, de prévenir et d'améliorer l'aspect lisse et les propriétés biomécaniques de la peau et de réduire les cernes chez les volontaires, et ceci de manière significative par rapport au groupe de volontaires traités avec la crème placebo.

La présente invention n'est pas destinée à être limitée dans sa portée aux modes de réalisation particulier décrits, qui sont fournis, par exemple, pour illustrer divers aspects de l'invention. Diverses modifications ou variantes des compositions et des procédés décrits deviendront évidentes au regard de la description et des enseignements fournis dans la présentes demandes. De telles modifications peuvent être réalisées sans s'écarter de la véritable portée et de l'esprit de la présente demande et sont destinées à être inclus dans la portée de la présente demande de brevet. Bien que l'invention puisse être décrite en relation avec des modes de réalisation préférés spécifiques, il faut comprendre que l'invention telle que revendiquée ne doit pas être indûment limitée à de tels modes de réalisation spécifiques. En effet, les différentes modifications ou variantes des modes de réalisation de l'invention qui sont évidentes pour l'homme du métier dans le domaine cosmétique ou des domaines apparentés sont destinées à entrer dans la portée des revendications suivantes.

**Revendications**

1. Utilisation cosmétique d'un extrait huileux de *Melaleuca alternifolia,* en tant qu'agent cosmétique régénérateur, réparateur ou anti-fatigue de la peau saine, dans laquelle ledit extrait comprend :

   - plus de 20% de composés choisis parmi les sesquiterpènes et sesquiterpénoïdes, et
   - moins de 6 % de terpinèn-4-ol,

   les pourcentages étant exprimés par rapport à la teneur totale de composés volatils présents dans l'extrait déterminée par chromatographie en phase gazeuse couplée à un détecteur à ionisation de flamme (GC/FID).

2. Utilisation cosmétique selon la revendication 1, **caractérisée en ce que** ledit extrait de *Melaleuca alternifolia* comprend moins de 15 %, de préférence moins de 10%, mieux encore moins de 2,0% de terpinéols.

3. Utilisation cosmétique selon la revendication 1 ou 2, **caractérisée en ce que** ledit extrait de *Melaleuca alternifolia* comprend :

   - moins de 5,0 %, de préférence moins de 2,0%, mieux moins de 1,0%, de terpinèn-4-ol, et/ou
   - moins de 5,0 %, de préférence moins de 2,0%, mieux moins de 1,5%, d'alpha-terpinéol, et/ou
   - plus de 30 %, de préférence plus de 40% de composés choisis parmi les sesquiterpènes et sesquiterpénoïdes.

4. Utilisation cosmétique selon l'une des revendications 1 à 3, **caractérisée en ce que** ledit extrait de *Melaleuca alternifolia* comprend :

   - de 5 à 15 % d'aromadendrène,

- de 10 à 25 % de lédène,
- de 10 à 25% de delta-cadinène,
- de 1 à 5 % de globulol, et
- de 1 à 5 % de viridifloral.

5. Utilisation cosmétique selon l'une des revendications 1 à 4, **caractérisée en ce que** ledit extrait de *Melaleuca alternifolia* est obtenu par distillation fractionnée d'une huile essentielle de feuilles et/ou de rameaux terminaux de *Melaleuca alternifolia.*

6. Utilisation cosmétique selon l'une des revendications 1 à 5, dans laquelle ledit extrait de *Melaleuca alternifolia* est utilisé pour traiter ou prévenir un ou plusieurs signes de la fatigue cutanée choisis parmi une perte d'éclat de la peau, un teint terne, un teint brouillé, une perte d'uniformité du teint, les traits du visage tirés, des cernes au niveau des yeux, des poches au niveau des yeux, les paupières gonflées, une altération de l'aspect lisse de la peau, une augmentation de la rugosité de la peau, une perte d'élasticité, une perte de densité, une perte de fermeté, une perte d'hydratation, une apparition de rides, une apparition de rougeurs, et leurs combinaisons.

7. Utilisation cosmétique selon l'une des revendications 1 à 5, dans laquelle ledit extrait de *Melaleuca alternifolia* est utilisé en tant qu'agent cosmétique pour favoriser ou améliorer la récupération cutanée pendant le sommeil et/ou en tant qu'agent cosmétique pour potentialiser les mécanismes naturels de réparation et de régénération de la peau contrôlés par la mélatonine.

8. Utilisation cosmétique selon l'une des revendications 1 à 7, dans laquelle ledit extrait de *Melaleuca alternifolia* est utilisé en outre pour augmenter la durée totale de sommeil, et/ou celle du sommeil profond.

9. Utilisation cosmétique selon l'une des revendications 1 à 8, dans laquelle ledit extrait de *Melaleuca alternifolia* est présent en tant qu'agent actif cosmétique dans une composition cosmétique, de préférence une crème, un baume, un masque, un sérum ou une lotion.

10. Extrait huileux de *Melaleuca alternifolia* comprenant

- plus de 20% de composés choisis parmi les sesquiterpènes et sesquiterpénoïdes, et
- moins de 6 % de terpinèn-4-ol,

les pourcentages étant exprimés par rapport à la teneur totale de composés volatils présents dans l'extrait déterminée par chromatographie en phase gazeuse couplée à un détecteur à ionisation de flamme (GC/FID), de préférence, ledit extrait huileux de *Melaleuca alternifolia* est tel que défini dans l'une quelconque des revendications 2 à 5.

11. Ingrédient cosmétique comprenant un extrait de *Melaleuca alternifolia* selon la revendication 10, de préférence comprenant de 0,1% à 1,0% en poids de l'extrait de *Melaleuca alternifolia* dans un véhicule cosmétiquement acceptable et, mieux encore, comprenant ou consistant essentiellement en 0,1% à 1,0% en poids de l'extrait *de Melaleuca alternifolia* dans un véhicule cosmétiquement acceptable choisi parmi le pentylène glycol ou la triheptanoïne.

12. Composition cosmétique comprenant l'extrait de *Melaleuca alternifolia* selon la revendication 10, ou l'ingrédient cosmétique selon la revendication 11, en combinaison avec au moins excipient acceptable sur le plan cosmétique.

13. Composition cosmétique selon la revendication 12, **caractérisée en ce qu'**elle comprend de 0,0005 à 0,01%, de préférence de 0,001 % à 0,005 % en poids dudit extrait de *Melaleuca alternifolia* ou de 0,5 % à 5 % en poids, de préférence de 1 % à 3% en poids, dudit ingrédient cosmétique.

14. Composition cosmétique selon la revendication 12 ou 13, ladite composition cosmétique étant choisie dans le groupe constitué par les solutions aqueuses, les solutions hydroalcooliques, les émulsions huile-dans-eau (H/E) ou eau-dans huile (E/H) ou multiple (triple : E/H/E ou H/E/H), les nanoémulsions, en particulier des nanoémulsions H/E, les gels aqueux, ou les dispersions d'une phase grasse dans une phase aqueuse à l'aide de sphérules, les suspensions, de préférence en milieux aqueux ou hydroalcoolique, les suspensions de liposomes, les poudres, les lotions, laits, les crèmes, les onguents, les gels, les mousses, et les pommades.

15. Composition cosmétique selon l'une des revendications 12 à 14, ladite composition étant un soin pour une utilisation dans le traitement ou la prévention de la fatigue cutanée, par exemple un sérum, une crème, un masque ou un baume, de préférence de nuit.

16. Procédé cosmétique pour prévenir, atténuer ou traiter les signes de la fatigue cutanée chez un individu, ledit procédé comprenant l'application d'une quantité cosmétiquement efficace d'un extrait de *Melaleuca alternifolia* selon la revendication 10, ou d'une composition cosmétique selon l'une des revendications 12 à 15 sur la peau.

**Patentansprüche**

1. Kosmetische Verwendung eines öligen Extrakts aus *Melaleuca alternifolia* als kosmetisches Mittel zur Regeneration, Reparatur oder Anti-Ermüdung gesunder Haut, wobei der Extrakt Folgendes umfasst:

   - mehr als 20 % Verbindungen, ausgewählt aus Sesquiterpenen und Sesquiterpenoiden, und
   - weniger als 6 % Terpinen-4-ol,

   wobei die Prozentangaben sich auf den Gesamtgehalt an flüchtigen Bestandteilen in dem Extrakts beziehen, der mittels Gaschromatographie gekoppelt mit einem Flammenionisationsdetektor (GC/FID) bestimmt wurde.

2. Kosmetische Verwendung nach Anspruch 1, **dadurch gekennzeichnet, dass** dieser Extrakt aus *Melaleuca alternifolia* weniger als 15 %, vorzugsweise weniger als 10 %, besonders bevorzugt weniger als 2,0 %, Terpineole enthält.

3. Kosmetische Verwendung nach Anspruch 1 oder 2, **dadurch gekennzeichnet, dass** dieser Extrakt aus *Melaleuca alternifolia* Folgendes enthält:

   - weniger als 5,0 %, vorzugsweise weniger als 2,0 %, besonders bevorzugt weniger als 1,0 %, Terpinen-4-ol und/oder
   - weniger als 5,0 %, vorzugsweise weniger als 2,0 %, besonders bevorzugt weniger als 1,5 %, $\alpha$-Terpineol und/oder
   - mehr als 30 %, vorzugsweise mehr als 40 %, Verbindungen, ausgewählt aus Sesquiterpenen und Sesquiterpenoiden.

4. Kosmetische Verwendung nach einem der Ansprüche 1 bis 3, **dadurch gekennzeichnet, dass** dieser Extrakt aus *Melaleuca alternifolia* Folgendes enthält:

   - 5 bis 15 % Aromadendren,
   - 10 bis 25 % Leden,
   - 10 bis 25 % Delta-Cadinen,
   - 1 bis 5 % Globulol und
   - 1 bis 5 % Viridifloral.

5. Kosmetische Verwendung nach einem der Ansprüche 1 bis 4, **dadurch gekennzeichnet, dass** der Extrakt aus *Melaleuca alternifolia* durch fraktionierte Destillation eines ätherischen Öls aus den Blättern und/oder Triebspitzen von *Melaleuca alternifolia* gewonnen wird.

6. Kosmetische Verwendung nach einem der Ansprüche 1 bis 5, wobei dieser Extrakt aus *Melaleuca alternifolia* zur Behandlung oder Vorbeugung eines oder mehrerer Anzeichen von Hautermüdung verwendet wird, ausgewählt aus, dem Verlust der Ausstrahlung der Haut, gelblichem Teint, ungleichmäßigem Hautton, eingefallenen Gesichtszügen, dunklen Augenringen, Tränensäcken, geschwollenen Augenlidern, veränderten Hautglätte, erhöhter Hautrauheit, Elastizitätsverlust, Dichteverlust, Festigkeitsverlust, Feuchtigkeitsverlust, Faltenbildung, Rötungen einer Zunahme der Hautrauigkeit, einem Elastizitätsverlust, einem Dichteverlust, einem Festigkeitsverlust, einem Feuchtigkeitsverlust, dem Auftreten von Falten, dem Auftreten von Rötungen und Kombinationen davon.

7. Kosmetische Verwendung nach einem der Ansprüche 1 bis 5, wobei dieser Extrakt aus *Melaleuca alternifolia* als kosmetischer Wirkstoff zur Förderung oder Verbesserung der Hautregeneration im Schlaf und/oder als kosmetischer Wirkstoff zur Verstärkung der durch Melatonin gesteuerten natürlichen Hautreparatur- und Regenerationsmechanismen verwendet wird.

8. Kosmetische Verwendung nach einem der Ansprüche 1 bis 7, wobei dieser Extrakt aus *Melaleuca alternifolia* weiterhin dazu verwendet wird, die Gesamtschlafzeit und/oder die Dauer des Tiefschlafs zu verlängern.

9. Kosmetische Verwendung nach einem der Ansprüche 1 bis 8, wobei dieser Extrakt aus *Melaleuca alternifolia* als kosmetischer Wirkstoff in einer kosmetischen Zusammensetzung, vorzugsweise einer Creme, einem Balsam, einer Maske, einem Serum oder einer Lotion, vorliegt.

10. Öliger Extrakt aus *Melaleuca alternifolia,* umfassend:

   - mehr als 20 % der Verbindungen ausgewählt aus Sesquiterpene und Sesquiterpenoide und
   - weniger als 6 % Terpinen-4-ol,

   wobei die Prozentangaben sich auf den Gesamtgehalt an flüchtigen Bestandteilen in dem Extrakts beziehen, der mittels Gaschromatographie gekoppelt mit einem Flammenionisationsdetektor (GC/FID) bestimmt wurde, wobei vorzugsweise dieser ölige Extrakt aus *Melaleuca alternifolia* der Definition in einem der Ansprüche 2 bis 5 entspricht.

11. Kosmetischer Inhaltsstoff, umfassend einen Extrakt aus *Melaleuca alternifolia* nach Anspruch 10, vorzugsweise umfassend 0,1 bis 1,0 Gew.-% des Extrakts aus *Melaleuca alternifolia* in einem kosmetisch verträglichen Trägerstoff und vorzugsweise umfassend oder bestehend im Wesentlichen aus 0,1 bis 1,0 Gew.-% des Extrakts aus *Melaleuca alternifolia* in einem kosmetisch verträglichen Trägerstoff, ausgewählt aus Pentylenglykol oder Triheptanoin.

12. Kosmetische Zusammensetzung, umfassend den Extrakt aus *Melaleuca alternifolia* nach Anspruch 10 oder den kosmetischen Inhaltsstoff nach Anspruch 11, in Kombination mit mindestens einem kosmetisch verträglichen Hilfsstoff.

13. Kosmetische Zusammensetzung nach Anspruch 12, **dadurch gekennzeichnet, dass** sie 0,0005 Gew.-% bis 0,01 Gew.-%, vorzugsweise 0,001 Gew.-% bis 0,005 Gew.-% dieses genannten Extrakts aus *Melaleuca alternifolia* oder 0,5 Gew.-% bis 5 Gew.-%, vorzugsweise 1 Gew.-% bis 3 Gew.-% dieses genannten kosmetischen Inhaltsstoffs enthält.

14. Kosmetische Zusammensetzung nach Anspruch 12 oder 13, wobei diese kosmetische Zusammensetzung ausgewählt ist aus der Gruppe bestehend aus wässrigen Lösungen, hydroalkoholischen Lösungen, Öl-in-Wasser (O/W)- oder Wasser-in-Öl (W/O)- oder Mehrfachemulsionen (Dreifach: O/W/O oder W/O/W), Nanoemulsionen, insbesondere O/W-Nanoemulsionen, wässrigen Gelen oder Dispersionen einer Ölphase in einer wässrigen Phase unter Verwendung von Sphärulen, Suspensionen, vorzugsweise in wässrigem oder hydroalkoholischem Medien, Liposomensuspensionen, Pulvern, Lotionen, Milchprodukten, Cremes, Salben, Gelen, Schäumen und Balsamen.

15. Kosmetische Zusammensetzung nach einem der Ansprüche 12 bis 14, wobei es sich bei dieser Zusammensetzung um ein Hautpflegeprodukt zur Verwendung in der Behandlung oder Vorbeugung von Hautermüdung handelt, beispielsweise ein Serum, eine Creme, eine Maske oder ein Balsam handelt, vorzugsweise zur Anwendung über Nacht.

16. Kosmetisches Verfahren zur Vorbeugung, Linderung oder Behandlung von Anzeichen von Hautermüdung in einem Individuum, wobei dieses Verfahren das Auftragen einer kosmetisch wirksamen Menge eines Extrakts aus *Melaleuca alternifolia* nach Anspruch 10 oder einer kosmetischen Zusammensetzung nach einem der Ansprüche 12 bis 15 auf die Haut umfasst.

**Claims**

1. A cosmetic use of an oily extract of *Melaleuca alternifolia,* as a regenerative, repairing or anti-fatigue cosmetic agent in a healthy skin, wherein said extract comprises:

   - more than 20% of compounds selected from sesquiterpenes and sesquiterpenoids, and
   - less than 6% of terpinen-4-ol,

   the percentages being expressed relative to the total content of volatile compounds present in the extract determined by gas chromatography coupled with a flame ionization detector (GC/FID).

2. The cosmetic use of claim 1, **characterized in that** said extract of *Melaleuca alternifolia* comprises less than 15%, preferably less than 10%, better still less than 2.0% of terpineols.

3. The cosmetic use of claim 1 or 2, **characterized in that** said extract of *Melaleuca alternifolia* comprises:

   - less than 5.0%, preferably less than 2.0%, better still less than 1.0%, of terpinen-4-ol, and/or
   - less than 5.0%, preferably less than 2.0%, better still less than 1.5%, of alpha-terpineol, and/or
   - more than 30%, preferably more than 40% of compounds selected from sesquiterpenes and sesquiterpenoids.

4. The cosmetic use of any one of claims 1 to 3, **characterized in that** said extract of *Melaleuca alternifolia* comprises:

   - from 5% to 15% of aromadendrene,
   - from 10% to 25% of ledene,
   - from 10% to 25% of delta-cadinene,
   - from 1% to 5% of globulol, and
   - from 1% to 5% of viridifloral.

5. The cosmetic use of any one of claims 1 to 4, **characterized in that** said extract of *Melaleuca alternifolia* is obtained by fractional distillation of an essential oil of leaves and/or terminal branches of *Melaleuca alternifolia.*

6. The cosmetic use of any one of claims 1 to 5, wherein said extract of *Melaleuca alternifolia* is used to treat or prevent one or more signs of skin fatigue selected from a loss of radiance of the skin, a dull complexion, a peaky complexion, a loss of uniformity of the complexion, haggard facial features, dark circles around the eyes, bags under the eyes, puffy eyelids, an alteration in the smooth appearance of the skin, an increase in the roughness of the skin, a loss of elasticity, a loss of density, a loss of firmness, a loss of hydration, an appearance of wrinkles, an appearance of redness, and combinations thereof.

7. The cosmetic use of any one of claims 1 to 5, wherein said extract of *Melaleuca alternifolia* is used as a cosmetic agent for promoting or improving skin recovery during sleep and/or as a cosmetic agent for potentiating the natural mechanisms of skin repair and regeneration controlled by melatonin.

8. The cosmetic use of any one of claims 1 to 7, wherein said extract of *Melaleuca alternifolia* is further used for increasing the total duration of sleep, and/or that of deep sleep.

9. The cosmetic use of any one of claims 1 to 8, wherein said extract of *Melaleuca alternifolia* is present as a cosmetic active agent in a cosmetic composition, preferably a cream, a balm, a mask, a serum or a lotion.

10. An oily extract of *Melaleuca alternifolia* comprising:

    - more than 20% of compounds selected from sesquiterpenes and sesquiterpenoids, and
    - less than 6% of terpinen-4-ol,

    the percentages being expressed relative to the total content of volatile compounds present in the extract determined by gas chromatography coupled with a flame ionization detector (GC/FID), preferably said oily extract of *Melaleuca alternifolia* is as defined in any one of claims 2 to 5.

11. A cosmetic ingredient comprising an extract of *Melaleuca alternifolia* of claim 10, preferably comprising from 0.1% to 1.0% by weight, or more preferably from 0.1% to 0.5% by weight, of the extract of *Melaleuca alternifolia* in a cosmetically acceptable carrier selected from pentylene glycol or triheptanoin.

12. A cosmetic composition comprising the extract of *Melaleuca alternifolia* of claim 10, or the cosmetic ingredient of claim 11, in combination with at least one cosmetically acceptable excipient.

13. The cosmetic composition of claim 12, **characterized in that** it comprises from 0.0005% to 0.01%, preferably from 0.001% to 0.005% by weight of said extract of *Melaleuca alternifolia* or from 0.5% to 5% by weight, preferably from 1% to 3% by weight, of said cosmetic ingredient.

14. The cosmetic composition of any one of claims 12 to 13, said cosmetic composition being selected from the group consisting of aqueous solutions, aqueous-alcoholic solutions, oil-in-water (O/W) emulsions or water-in-oil (W/O) emulsions or multiple (triple: W/O/W or O/W/O) emulsions, nanoemulsions, in particular O/W nanoemulsions, aqueous gels, or spherule-assisted dispersions of a fatty phase in an aqueous phase, suspensions, preferably in aqueous or aqueous-alcoholic media, liposome suspensions, powders, lotions, milks, creams, unguents, gels, foams, and ointments.

15. The cosmetic composition of any one of claims 12 to 14, said composition being a care product for use in the treatment or prevention of skin fatigue, for example a serum, a cream, a mask or a balm, preferably for use at night.

16. A cosmetic method for preventing, reducing or treating signs of skin fatigue in subject, said method comprising the application of a cosmetically effective amount of an extract of *Melaleuca alternifolia* of claim 10, or that of a cosmetic composition of any one of claims 12 to 15 on the skin.

**Figure 1**

**Figure 2**

**Figure 3**

**Figure 4**

Figure 5

Figure 6

Figure 7

Figure 8

Figure 9

Figure 10

**RÉFÉRENCES CITÉES DANS LA DESCRIPTION**

**Documents brevets cités dans la description**

- FR 2748204